# FASCICULE DE BREVET EUROPEEN

(11) **EP 0 774 004 B1**
(45) Date de publication et mention de la délivrance du brevet: **20.09.2006**
(21) Numéro de dépôt: 95927005.9
(22) Date de dépôt: 01.08.1995
(51) Int. Cl.: C12N 15/57, C12N 9/64, C07K 16/40, A61K 38/48, C12N 5/10

(54) **SEQUENCES D'ADN CODANT POUR LES PROTEINES HUMAINES TX ET TY APPARENTEES A L'ENZYME DE CONVERSION DE L'INTERLEUKINE-1BETA**
DNA-SEQUENZEN, DIE FÜR DIE MENSCHLICHE PROTEINE Tx UND Ty KODIEREN, DIE ICE GLEICHEN
DNA SEQUENCES CODING FOR THE HUMAN PROTEINS TX AND TY RELATED TO THE INTERLEUKIN-1BETA CONVERTING ENZYME

(30) Priorité: 02.08.1994 FR 9409567
(43) Date de publication de la demande: 21.05.1997
(73) Titulaire: Aventis Pharma S.A., 92160 Antony (FR)
(72) Inventeur: DIU, Anita, F-94220 Charenton-le-Pont (FR); FAUCHEU, Chi, F-77184 Emervainville (FR); HERCEND, Thierry, F-94220 Charenton-le-Pont (FR); LALANNE, Jean-Louis, F-94120 Fontenay-sous-Bois (FR); LIVINGSTON, David, J., Newton, MA 02160 (US); SU, Michael, S.-S., Newton, MA 02159 (US)
(86) Numéro de dépôt international: PCT/FR1995/001035
(87) Numéro de publication internationale: WO 1996/004387

(56) Documents cités:
- WO-A1-95/27792
- WO-A1-95/27793
- WO-A1-96/00297
- CELL, vol. 75, 19 Novembre 1993 pages 641-652, J. YUAN ET AL 'The C. elegans cell death gene ced-3 encodes a protein similar to mammalian interleukin-1beta converting enzyme' cité dans la demande
- GENES DEV. (1994), 8(14), 1613-26 CODEN: GEDEEP;ISSN: 0890-9369, KUMAR, SHARAD ET AL 'Induction of apoptosis by the mouse Nedd2 gene, which encodes a protein similar to the product of the Caenorhabditis elegans cell death gene ced-3 and the mammalian IL-1.beta.- converting enzyme'
- JOURNAL OF BIOLOGICAL CHEMISTRY, vol. 269, no. 49, 9 Décembre 1994 MD US, pages 30761-30764, T. FERNANDES-ALNEMRI ET AL 'CCP32, a novel human apoptotic protein with homology to Caenorhabditis elegans cell death protein Ced-3 and mammalian Interleukin-1-beta-converting enzyme'
- JOURNAL OF BIOLOGICAL CHEMISTRY 270 (26). 1995. 15870-15876. ISSN: 0021-9258, 30 Juin 1995 MUNDAY N A ET AL 'Molecular Cloning and Pro-apoptotic Activity of ICE-relII and ICE-relIII, Members of the ICE-CED-3 Family of Cysteine Proteases.'
- EMBO (EUROPEAN MOLECULAR BIOLOGY ORGANIZATION) JOURNAL 14 (9). 1995. 1914-1922. ISSN: 0261-4189, 1 Mai 1995 FAUCHEU C ET AL 'A novel human protease similar to the interleukin-1-beta converting enzyme induces apoptosis in transfected cells.'
- FAUCHEU C. ET AL: 'Identification of a cysteine protease closely related to interleukin-1beta-converting enzyme' EUR.J.BIOCHEM. vol. 236, 1996, pages 207 - 213, XP002930916
- FASSY F. ET AL: 'Enzymatic activity of two caspases related to interleukin-1beta-converting enzyme' EUR.J.BIOCHEM. vol. 253, 1998, pages 76 - 83
- MARTINON F.; TSCHOPP J.: '"Inflammatory caspases: linking an intracellular innate immune system to autoinflammatory diseases' CELL vol. 117, 2004, pages 561 - 574

## Description

La présente invention concerne une séquence d'ADN codant pour une nouvelle protéine humaine Tx apparentée à l'enzyme de conversion de l'interleukine-1béta, la protéine Tx, leur procédé de production, les compositions pharmaceutiques la contenant et leurs applications comme médicaments.

L'interleukine-1béta (IL-1β) est une cytokine pro-inflammatoire impliquée dans la pathogénie de multiples maladies inflammatoires aiguës ou chroniques telles que la polyarthrite rhumatoïde, les maladies inflammatoires des intestins ou le choc septique (Dinarello et al., 1992, Immunological Reviews, 127, 119-146).

Les monocytes et les macrophages humains synthétisent l'IL-1β sous forme d'un précurseur inactif de 31kDa (pIL-1β). Le pIL-1β ne possède pas de séquence signal conventionnelle et ne peut être secrété efficacement par la cellule qu'après coupure entre l'acide aspartique 116 et l'alanine 117. Cette coupure, qui génère la forme IL-1β active de 17kDa, est effectuée par une enzyme spécifique appelée interleukine-1béta converting enzyme (ICE) (Thornberry et al., 1992, Nature, 356, 768-774 ; Cerretti et al., 1992, Science, 256, 97-100). Cette enzyme a été caractérisée et clonée chez l'homme et chez la souris (Nett et al., 1992, Journal of Immunology, 149, 3254-3259 ; Molineaux et al., 1993, Proc. Natl. Acad. Sci. USA, 90, 1809-1813). C'est une cystéine protéase unique qui ne présente pas d'homologie avec les autres thiol-protéases connues. Elle possède également une spécificité particulière pour certaines liaisons peptidiques Asp-X de la pIL-1β.

L'enzyme ICE est composée de deux sous-unités de 20kDa (p20) et 10kDa (p10) dont l'association est nécessaire à l'activité enzymatique. Ces sous-unités proviennent du clivage protéolytique d'une forme pro-enzyme de 45kDa (p45). L'enzyme ICE elle-même est capable de cliver son précurseur p45 ou la forme p30 de 30 kDa, qui ne possède pas les 119 acides-aminés de la partie N-terminale de la pro-enzyme, en forme p20 plus p10 active. La séquence complète de p45 a été caractérisée par son ADNc ainsi que la séquence en acides aminés (Thornberry et al. déjà cité). La caractérisation du gène de l'ICE humain a été décrite (Cerretti et al., 1994, Genomics, 20, 468-473).

Des travaux récents ont mis en évidence un rôle possible de l'ICE dans la régulation de la mort cellulaire programmée ou apoptose (Yuan et al., 1993, Cell, 75, 641-652). En effet, l'ICE présente une homologie de 28 % avec Ced-3, une protéine de C. elegans impliquée dans l'apoptose, et la surexpression de l'ICE murine dans des fibroblastes de rat déclenche l'apoptose (Miura et al., 1993, Cell, 75,653-660). De plus, l'expression de la protéine crmA, une serpine virale inhibitrice de l'ICE, dans des neurones ganglionnaires de poulet transfectés protège ces cellules de la mort par apoptose induite par la suppression de facteur de croissance (nerve growth factor) (Gagliardini et al., 1994, Science, 263, 826-828). Ces observations suggèrent que l'ICE ou des homologues de cette protéine pourraient être impliqués dans la régulation de la mort cellulaire programmée observée notamment dans les maladies neuronales dégénératives telles que la maladie d'Alzheimer ou la maladie de Parkinson ainsi que dans les ischémies cérébrales (Barinaga, M, Science, 259, 762, 1993).

La mise en évidence de nouvelles protéines apparentées à l'ICE jouant un rôle soit dans la maturation de l'IL-1β soit dans l'apoptose peut contribuer au développement de nouveaux agents thérapeutiques ou diagnostiques dans des situations dans lesquelles l'IL-1β ou l'apoptose sont impliquées.

Une protéine similaire à l'IL-1beta converting enzyme (ICE) est encodée par le gène Nedd2 de la souris (Kumar et al., Genes & Dev. 8:1613-1626, 1994).

Des cystéine-protéases de la même famille ont été décrites après la date de priorité de la présente demande de brevet (Munday et al., J.Biol.Chem 270;15870-15876, numéro du 30 juin 1995).

La présente invention concerne une nouvelle protéine humaine Tx qui présente une homologie de 52 % environ avec le précurseur humain p45 de l'ICE et qui ne permet pas la maturation du précurseur de l'IL-1β en cytokine active. La protéine Tx possède deux fonctions inattendues : d'une part, c'est une protéase et elle est notamment capable de cliver le précurseur p30 de l'ICE en sous-unités p10 et p20 et d'autre part, elle est capable d'induire l'apoptose dans des cellules, par exemple dans des cellules Cos transfectées.

Ces propriétés biologiques permettent de prévoir l'utilisation de la protéine Tx dans le traitement de situations pathologiques qui répondent à l'IL-1β ou dans lesquelles l'apotose intervient.

La présente invention concerne aussi une nouvelle protéine humaine Ty qui présente une homologie supérieure à 70 % avec la protéine Tx. La protéine Ty est capable d'induire l'apoptose dans des cellules, par exemple des cellules Cos transfectées. La protéine Ty est une protéase qui est capable de s'autocliver de façon intermoléculaire.

La présente invention a donc pour objet une séquence d'ADN codant pour un polypeptide humain ayant une activité protéase et ayant la séquence nucléotidique de la séquence SEQ ID N° 1 : ainsi qu'une séquence d'ADN codant pour un polypeptide humain ayant la capacité d'induire l'apoptose et ayant la séquence nucléotidique de la séquence SEQ ID N° 1 ci-dessus.

L'invention a particulièrement pour objet une séquence d'ADN codant pour un polypeptide humain ayant une activité protéase et capable d'induire l'apoptose et ayant la séquence nucléotidique de la séquence SEQ ID N° 1 ci-dessus.

La séquence d'ADN ci-dessus qui code pour une protéine ayant 377 acides aminés est une séquence d'ADNc qui peut être obtenue par amplification par PCR, à partir d'ARN de monocytes activés par le LPS ou de polynucléaires ou de placenta humains, grâce à des oligonucléotides dérivés de la séquence de l'ICE et de la séquence de gènes homologues préalablement identifiés, selon des conditions opératoires dont une description détaillée est donnée plus loin.

La mise en évidence de l'activité protéase ainsi que celle de la capacité à induire l'apoptose sont illustrées plus loin dans la partie expérimentale.

Le brevet décrit notamment une séquence d'ADN codant pour un polypeptide humain ayant une activité protéase et capable d'induire l'apoptose ayant la séquence commençant au nucléotide 42 et se terminant au nucléotide 1172 de la séquence SEQ ID N° 1 ainsi que les séquences d'ADN qui hybrident avec celle-ci et ayant la même fonction.

Par séquences qui hybrident, on inclut les séquences d'ADN qui hybrident sous des conditions de forte stringence et qui codent pour un polypeptide ayant la même activité. Les conditions de stringence comprennent par exemple une hybridation à 65°C, pendant 18 heures dans une solution 5X SSPE ; 10X Denhardt ; 100 µg/ml DNAss ; 1 % SDS suivie de 3 lavages pendant 5 minutes avec 2X SSC ; 0,05 % SDS, puis 3 lavages pendant 15 minutes à 65°C dans 1X SSC ; 0,1 % SDS, selon Maniatis et al., Molecular cloning, Cold Spring Harbor Laboratory Press, 1989.

La connaissance de la séquence SEQ ID N° 1 permet de reproduire la présente invention par exemple par les méthodes connues de synthèse chimique ou par criblage d'une banque génomique ou d'une banque d'ADNc à l'aide de sondes d'oligonucléotides de synthèse par les techniques connues d'hybridation.

L'invention concerne aussi un polypeptide humain ayant une activité protéase et capable d'induire l'apoptose et ayant la séquence en acides aminés de la séquence SEQ ID N° 2 ainsi que les allèles et les analogues de cette séquence.

Par allèles et analogues, on inclut les séquences modifiées par substitution, délétion ou addition d'un ou plusieurs acides aminés pour autant que ces produits conservent la même fonction.

L'invention a spécialement pour objet le polypeptide ayant la séquence en acides aminés de la séquence SEQ ID N° 2 et désigné protéine Tx.

Un des aspects de l'invention concerne également un polypeptide selon l'invention tel qu'obtenu par l'expression dans une cellule hôte d'un ADN codant pour la séquence en acides aminés de la séquence SEQ ID N° 2.

Lorsque le polypeptide de l'invention est obtenu par expression dans une cellule hôte, celle-ci est réalisée selon les méthodes connues de génie génétique et de culture cellulaire.

L'expression peut être réalisée dans une cellule procaryote, par exemple E. coli ou dans une cellule eucaryote, par exemple une cellule Cos contenant la séquence d'ADN codant pour le polypeptide de l'invention précédée d'une séquence promoteur convenable.

L'invention concerne notamment un polypeptide selon l'invention tel qu'obtenu par l'expression dans une cellule hôte eucaryote.

L'invention concerne tout spécialement un polypeptide selon l'invention dont l'activité protéase correspond à la capacité de maturer l'enzyme de conversion de l'IL-1béta. Un exemple de détermination de cette activité protéase particulière est décrit plus loin.

L'invention a aussi pour objet un vecteur d'expression comprenant une séquence d'ADN codant pour un polypeptide humain ayant une activité protéase et capable d'induire l'apoptose ainsi qu'une cellule hôte transformée par un vecteur ci-dessus.

Les vecteurs d'expression sont des vecteurs connus permettant l'expression de la protéine sous le contrôle d'un promoteur convenable. Pour les cellules procaryotes, le promoteur peut être par exemple le promoteur lac, le promoteur trp, le promoteur tac, le promoteur β-lactamase ou le promoteur PL. Pour les cellules de levure, le promoteur peut être par exemple le promoteur PGK ou le promoteur AD. Pour les cellules de mammifères, le promoteur peut être par exemple le promoteur SV40 ou les promoteurs de l'adénovirus. Des vecteurs type Baculovirus peuvent être aussi utilisés pour l'expression dans des cellules d'insectes.

Les cellules hôtes sont par exemple des cellules procaryotes ou des cellules eucaryotes. Les cellules procaryotes sont par exemple E. coli, Bacillus ou Streptomyces. Les cellules hôtes eucaryotes comprennent des levures ainsi que des cellules d'organismes supérieurs, par exemple des cellules de mammifères ou des cellules d'insectes. Les cellules de mammifères sont par exemple des fibroblastes tels que des cellules CHO ou BHK de hamster et des cellules Cos de singe. Les cellules d'insectes sont par exemple des cellules SF9.

L'invention concerne un procédé qui comprend l'expression de la protéine Tx dans une cellule hôte transformée par un ADN codant pour la séquence en acides aminés de la séquence SEQ ID N° 2 et notamment un procédé dans lequel la cellule hôte est une cellule eucaryote.

Le brevet décrit aussi des anticorps dirigés contre le polypeptide selon l'invention.

Les anticorps polyclonaux ou monoclonaux de l'invention peuvent être préparés selon les méthodes connues et peuvent être utilisés par exemple pour le dosage de la protéine Tx, par exemple dans un test ELISA et comme agents de diagnostic.

La nouvelle protéine Tx de l'invention a de remarquables propriétés biologiques, en particulier une activité protéase, notamment la capacité à maturer l'enzyme de conversion de l'IL-1béta ainsi que la capacité à induire l'apotose, comme le montrent les résultats donnés plus loin.

Ces propriétés biologiques rendent la protéine Tx de l'invention utilisable par exemple dans le traitement des maladies autoimmunes, dans la cicatrisation des plaies ou dans la réduction des effets secondaires aux traitements par irradiation dans lesquels l'Il-1β est impliquée ou par exemple dans le domaine des cancers et de l'infection dans lesquels l'apoptose intervient.

La présente invention a donc pour objet, à titre de médicament, le polypeptide selon l'invention.

L'invention s'étend aux compositions pharmaceutiques renfermant comme principe actif un médicament défini ci-dessus et concerne particulièrement les compositions pharmaceutiques pour moduler la production d'IL-1 béta ou pour moduler l'apoptose.

Le principe actif peut être incorporé à des excipients usuels pour la préparation des compositions pharmaceutiques ci-dessus. Les compositions peuvent être administrées par voie parentérale, orale ou locale.

Les polypeptides de l'invention permettent aussi d'envisager de nouveaux agents thérapeutiques constitués d'inhibiteurs de ces polypeptides et leur utilisation comme médicament, par exemple dans le traitement de l'inflammation associée à des maladies autoimmunes, le choc septique ou les maladies neurodégénératives.

L'invention concerne aussi une séquence d'ADN hybridant avec la séquence d'ADN commençant au nucléotide 42 et se terminant au nucléotide 1172 de la séquence SEQ ID N° 1 et ayant la même fonction.

L'hybridation est obtenue par exemple dans un tampon 5xSSC, 10xDenhardt, 100 microg/ml ADN de sperme de saumon, 1 % SDS pendant une nuit à 65°C. Les lavages sont ensuite effectués par exemple dans un tampon 1xSSC, 0,1 % SDS, deux fois 30 mn à 60°C.

L'invention concerne particulièrement la séquence d'ADN codant pour un polypeptide ayant une activité protéase et capable d'induire l'apoptose et ayant la séquence nucléotidique de la séquence SEQ ID N° 22 et plus particulièrement la séquence commençant au nucléotide 104 et se terminant au nucléotide 1195 de la séquence SEQ ID N° 22.

La séquence d'ADN SEQ ID N° 22 ci-dessus, qui code pour une protéine ayant 364 acides aminés, est une séquence d'ADNc qui peut être obtenue, par exemple, par amplification par PCR, à partir d'ADNc de rate ou de placenta humains, grâce à des oligonucléotides dérivés de la séquence ADNc Tx (SEQ ID N° 1). Un exemple détaillé de préparation est donné plus loin dans la partie expérimentale. La connaissance de la séquence SEQ ID N° 22 permet de reproduire la présente invention par exemple par les méthodes connues de synthèse chimique ou de criblage de banques génomiques ou de banques d'ADNc à l'aide de sondes d'oligonucléotides par les techniques d'hybridation.

L'invention a également pour objet un polypeptide humain ayant une activité protéase et capable d'induire l'apoptose et ayant la séquence en acides aminés de la séquence SEQ ID N° 23 et désigné protéine Ty.

Un des aspect de l'invention concerne également un polypeptide selon l'invention tel qu'obtenu par l'expression dans une cellule hôte d'un ADN codant pour la séquence en acides aminés de la séquence SEQ ID N° 23.

L'invention concerne aussi les cellules hôtes, les vecteurs d'expression qui permettent d'obtenir l'expression de la protéine Ty et dont des exemples ont été indiqués ci-dessus pour l'expression de la protéine Tx.

L'invention concerne aussi un procédé qui comprend l'expression de la protéine Ty dans une cellule hôte transformée par un ADN codant pour la séquence en acides aminés de la séquence SEQ ID N° 23.

L'invention concerne aussi les anticorps polyclonaux ou les anticorps monoclonaux dirigés contre la protéine Ty.

L'invention concerne aussi les compositions pharmaceutiques renfermant la protéine Ty à titre de médicament.

Les figures ci-annexées illustrent certains aspects de l'invention :

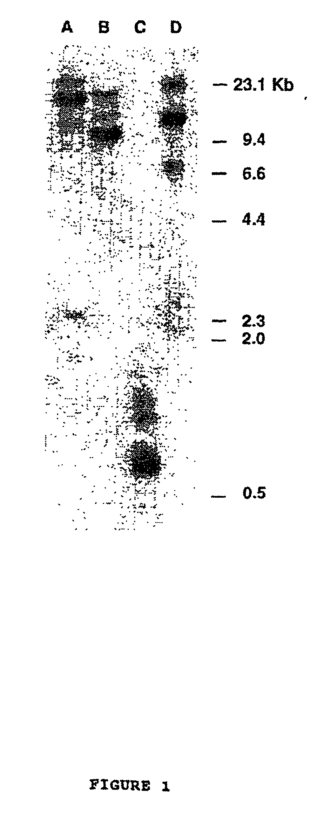

La figure 1 représente la détection de séquences homologues à l'ICE par Southern Blot dans l'ADN génomique humain issu de PBMC digéré par les enzymes de restriction BglII (ligne A) ; PstI (ligne B) ; HindIII (ligne C) ; BamHl (ligne D). La détection est faite par hybridation avec une sonde ICE exon 6 marquée au ³²P.

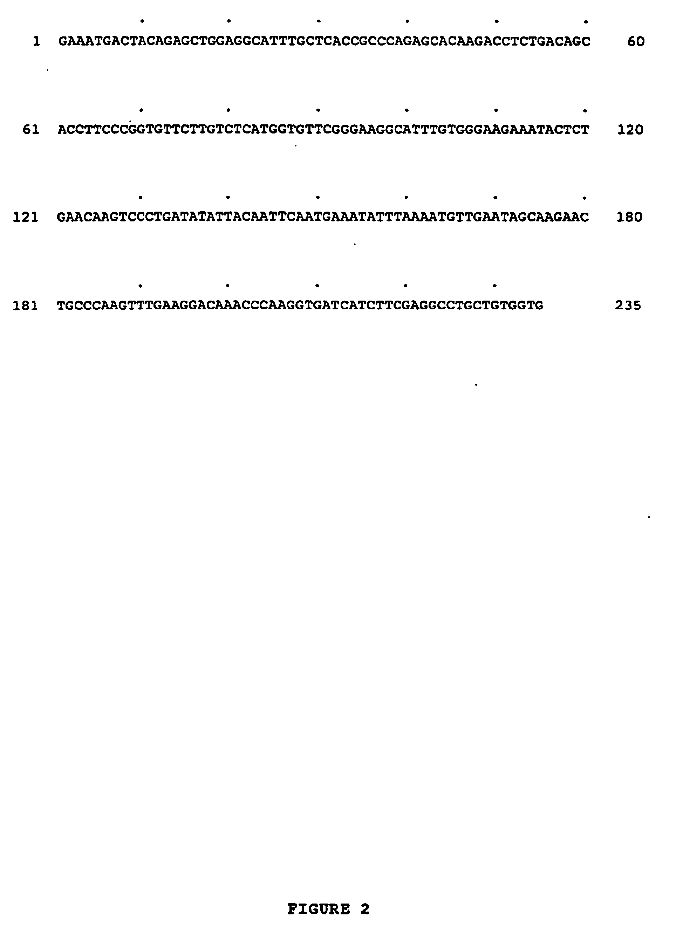

La figure 2 représente la séquence nucléotidique de l'exon 6 du gène T2 (SEQ ID N° 5).

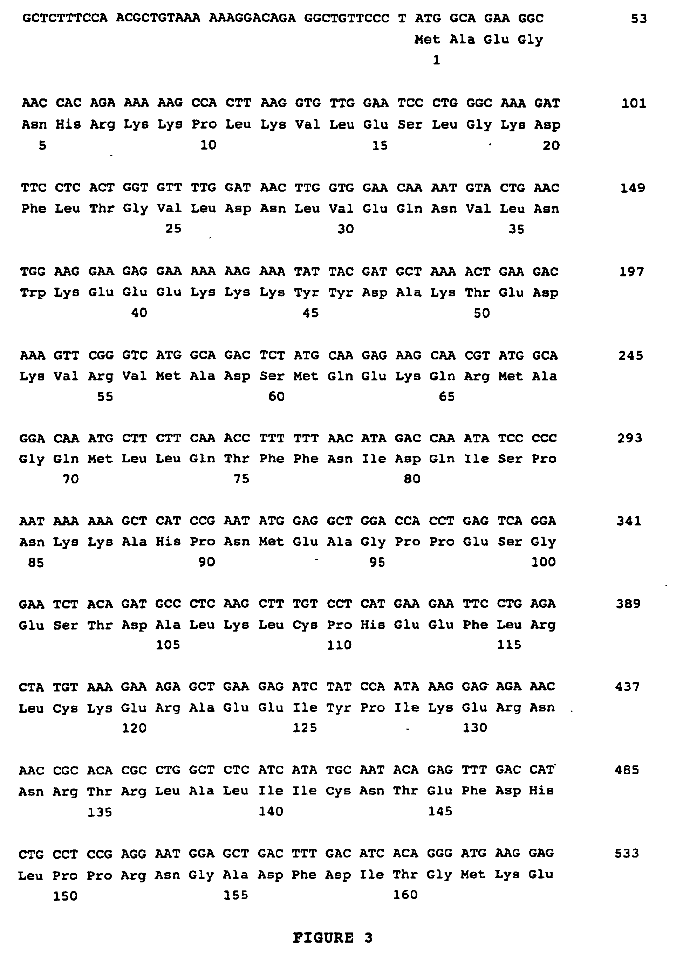

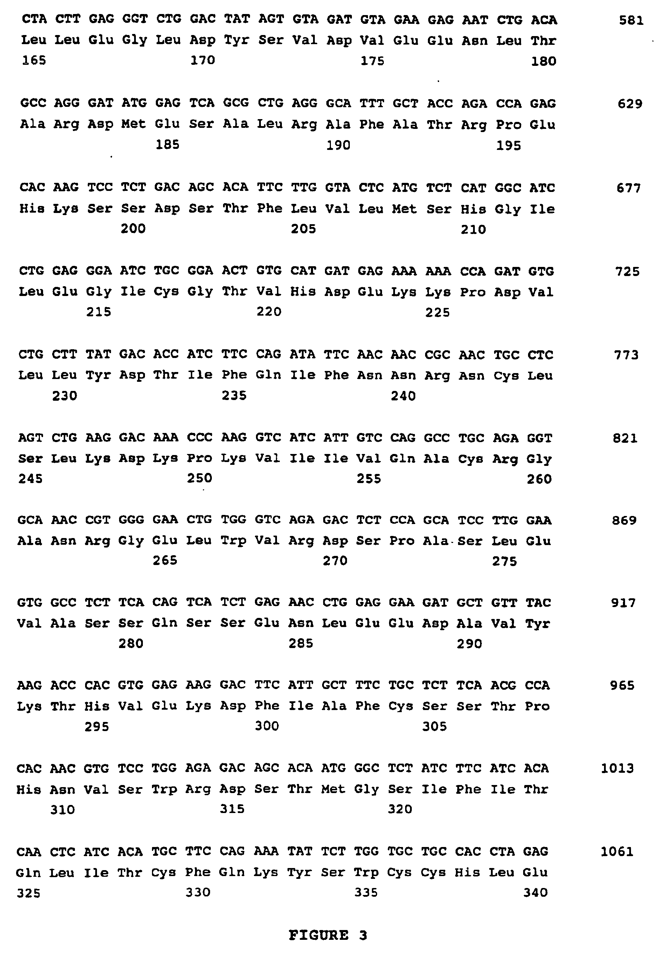

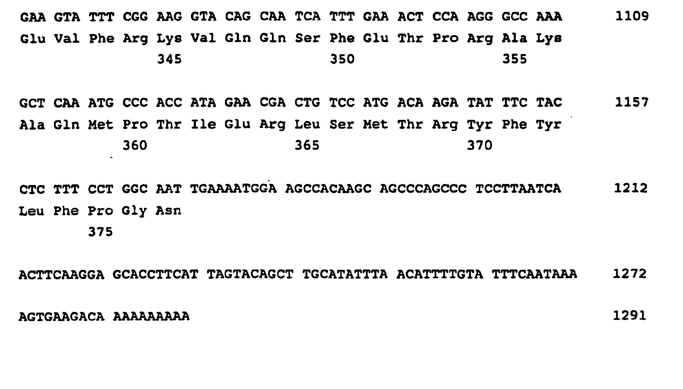

La figure 3 représente la séquence nucléotidique de l'ADNc Tx (SEQ ID N° 1) et la séquence en acides aminés correspondante (SEQ ID N° 2).

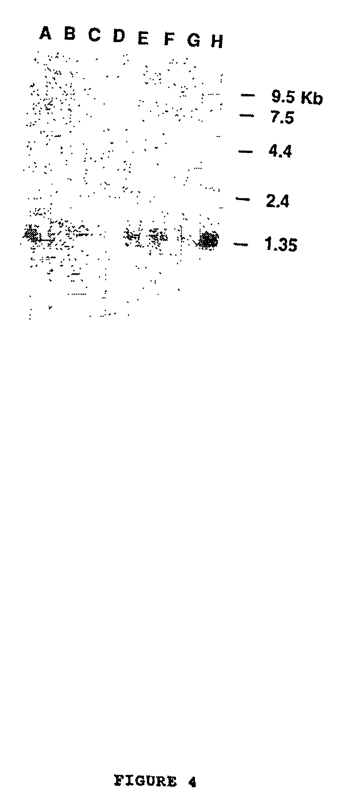

La figure 4 représente la détection d'ARNm Tx par Northern Blot dans les tissus de la rate (ligne A) ; du thymus (ligne B) ; de la prostate (ligne C) ; du testicule (ligne D) ; de l'ovaire (ligne E) ; de l'intestin grèle (ligne F) ; du colon (ligne G) ; des leucocytes périphériques (ligne H). La détection est faite par hybridation avec une sonde "Tx exon 6" marquée au ³²P.

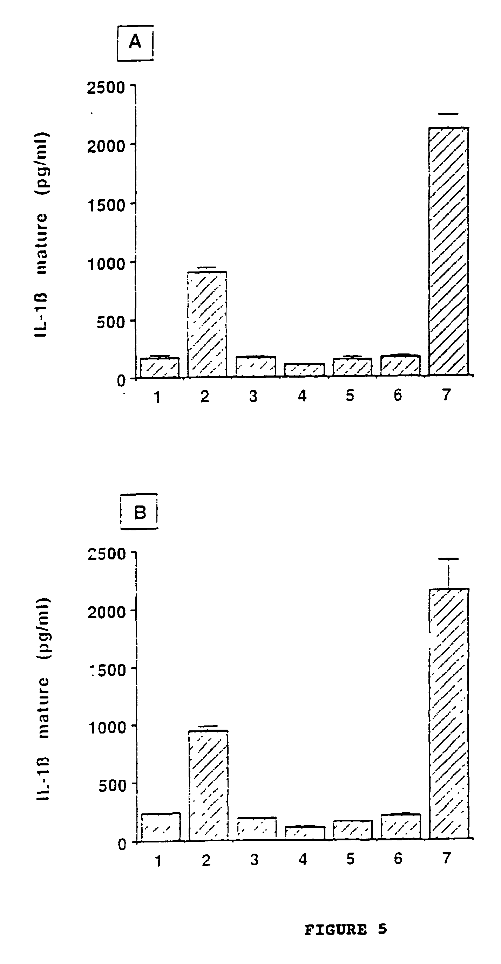

La figure 5 représente la sécrétion de l'IL-1β mature dans des cellules Cos-1 contenant constitutivement le pIL-1β et transfectées avec le vecteur pcDL-SRα296 contenant ICE p45 (ligne 2) ou Tx (ligne 3), le vecteur pcDL-SRα296 seul (ligne 4), le vecteur pcDNAI/Amp seul (ligne 5), le vecteur pcDNAI/Amp contenant Tx (ligne 6) ou ICE p30 (ligne 7) comparativement à une culture contrôle sans ADN (ligne 1). L'IL-1β mature est mesurée en pg/ml de surnageant cellulaire par ELISA (A : incubation 16 heures ; B : incubation 24 heures).

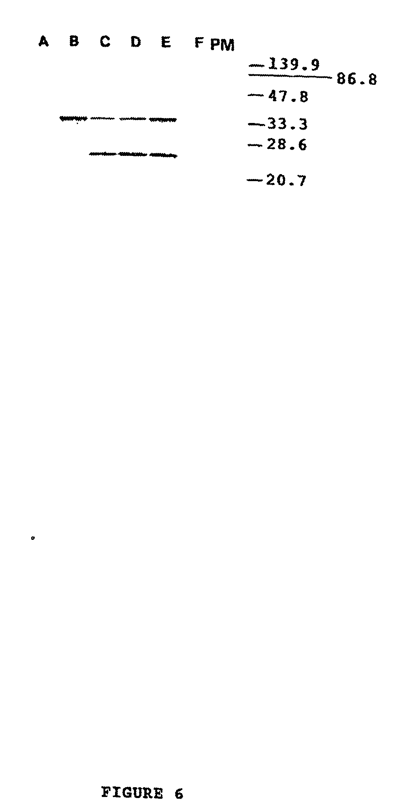

La figure 6 représente le clivage du précurseur ICE p30 dans des cellules Cos-1 transfectées par le vecteur pcDL-SRα296 seul (ligne A) ou contenant le mutant marqué T7-ICEp30C285S (ligne B) ou cotransfectées avec le vecteur pcDL-SRα296 contenant le mutant marqué T7-ICEp30C285S et le vecteur contenant ICE p30 (ligne C) ou ICE p45 (ligne D) ou Tx (ligne E). La détection est faite par Western Blot avec l'anticorps anti-T7 avec un contrôle correspondant à une transfection par le vecteur pcDL-SRα296 contenant Tx seul (ligne F).

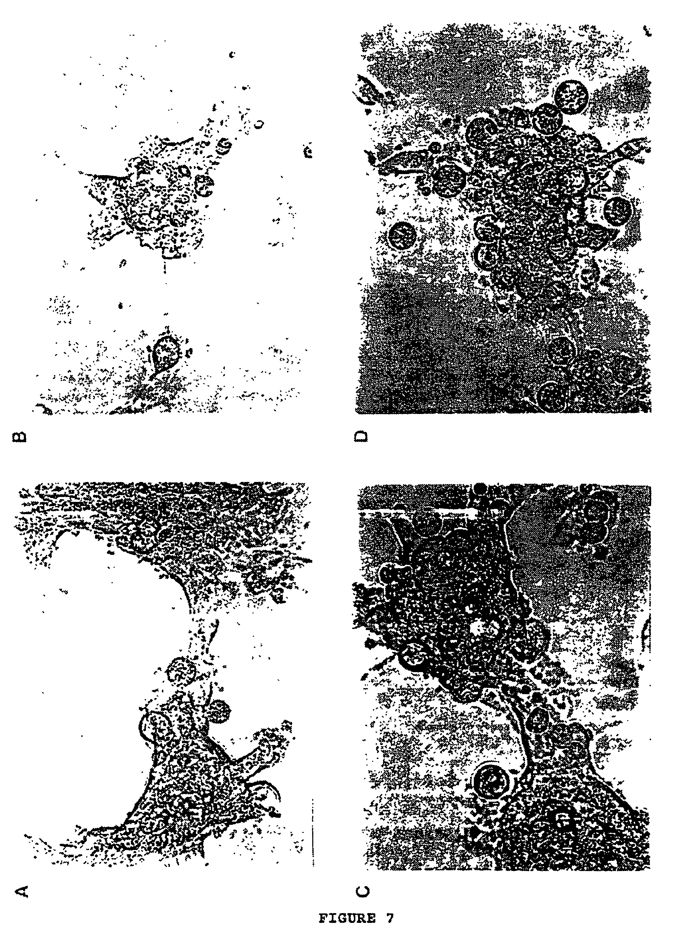

La figure 7 représente l'induction de l'apoptose par la protéine Tx dans des cellules Cos-1 transfectées par le vecteur pcDL-SRα296 seul (7B) ou contenant Tx (7C) ou ICE p45 (7D) comparativement à une culture contrôle sans ADN (7A) après une culture de 22 heures (grossissement x 400).

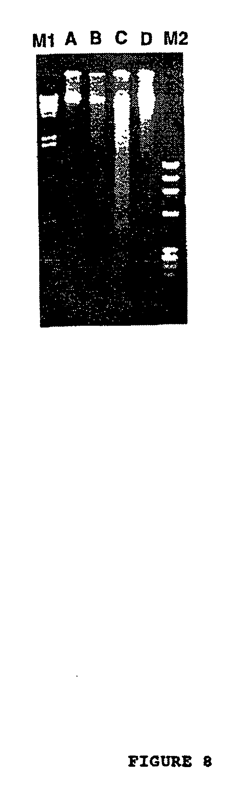

La figure 8 représente l'ADN des cellules Cos-1 transfectées par le vecteur pcDL-SRα296 contenant ICE p45 (ligne B) ou Tx (ligne C) ou par le vecteur seul (ligne D) comparativement à une culture contrôle sans ADN (ligne A). La détection est faite par coloration avec le BET après migration sur un gel d'agarose avec des marqueurs de taille ADN de phage lambda digéré par HindIII (Ml) et ADN de phage ΦX174 digéré par HindIII (M2).

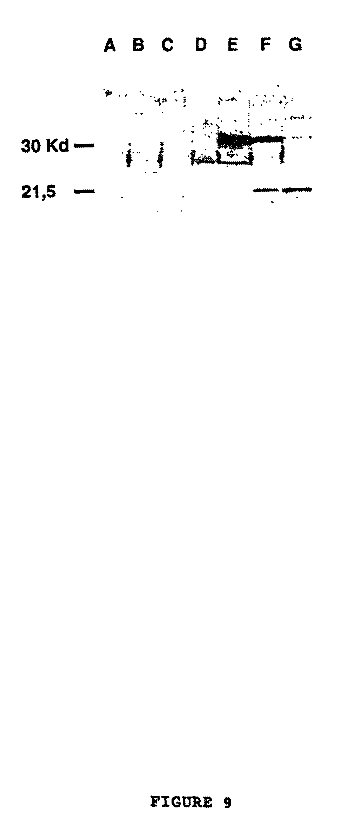

La figure 9 représente le clivage de la protéine Ty mutée (T7TYΔ67C245S) dans des cellules Cos-1, soit transfectées par le vecteur pcDL-SRα296 seul (ligne B) ou le vecteur pcDNAI/Amp seul (ligne C) ou le vecteur pT7TYΔ67C245S (ligne E), soit co-transfectées par le vecteur pT7TY et le vecteur pT7TYΔ67C245S (ligne F ; 23 heures et ligne G ; 43 heures). La détection est faite par Western Blot comparativement à une culture contrôle sans ADN (ligne A) et des marqueurs de poids moléculaires (ligne D ; non détectables).

Les exemples suivants illustrent l'invention sans la limiter.

### Exemple 1 : Identification de la séquence Tx

### A - Mise en évidence de gènes homologues à l'ICE humaine

Des gènes homologues à l'ICE ont été identifiés par Southern Blot en utilisant une sonde d'ADN correspondant à l'exon 6 du gène de l'ICE.

### a) Préparation de la sonde ICE exon 6

Les limites de l'exon 6 de l'ICE humain ont été décrites ainsi que l'organisation intron/exon complète du gène ICE humain (Cerretti et al. déjà cité). L'exon 6 (235 pb) correspond aux nucléotides 635 à 868 de la séquence ADNc p45 de l'ICE décrite (Thornberry et al. déjà cité).

La sonde ICE exon 6 a été préparée par amplification par PCR avec les oligonucléotides suivants :

| | | |
|---|---|---|
| ICE 6.5 : | ACATGACTAC AGAGCTGGAG | (SEQ ID N° 3) |
| ICE 6.3 : | CACCACGGCA GGCCTGGATG | (SEQ ID N° 4) |

qui ont été choisis en utilisant les données publiées (Thornberry et al. déjà cité), synthétisés et utilisés pour amplifier par RT-PCR l'ARN provenant de monocytes sanguins humains, extrait et purifié à l'aide d'une trousse RNA^{™} (Bioprobe), en utilisant les conditions d'amplification suivantes : Enzyme Biotaq (BioProbe) ; 30 cycles (94°C, 1mn ; 60°C, 1mn ; 72°C, lmn) ; Appareil PCR : Perkin-Elmer (GeneAmp PCRSystem 9600).

L'ADN exon 6 obtenu a été purifié par centrifugation sur colonne Spin X (Costar) et marqué au ³²P par la technique d'amorçage aléatoire ("random priming") au moyen de la trousse oligolabelling kit (Pharmacia Biotech).

### b) Hybridation à l'ADN génomique : Southern Blot

La sonde ICE exon 6 radiomarquée obtenue a été utilisée comme sonde d'hybridation sur un ADN génomique humain.

L'ADN génomique humain a été préparé à partir de cellules mononucléées du sang périphérique (PBMC) avec la trousse TurboGen (Invitrogen) puis coupé respectivement par les enzymes de restriction BglII, PstI, HindIII ou BamHI (Boehringer Mannheim), migré sur un gel d'agarose 0,9 % en 1x TAE, transféré sur une membrane de nylon GeneScreen Plus (NEN Dupont) puis hybridé avec la sonde ICE exon 6.

Les conditions d'hybridation sont celles décrites par Maniatis et al. (déjà cité) réalisées dans 5x SSPE, 10x Denhart, 100 µg/ml DNAss, 1 % SDS, une nuit à 65°C suivie de lavages réalisés successivement dans 2x SSC, 0,05 % SDS, 30 mn à température ambiante puis 1x SSC, 0,1 % SDS, 30 mn à 65°C, ce qui correspond à une forte stringence. Le tampon de lavage est préparé à partir des solutions stocks suivantes
- 20x SSC : solution aqueuse 3M Chlorure de Sodium, 0,3 M Citrate de Sodium
- 10 % SDS : solution aqueuse de Dodécyl Sulfate de Sodium, Après lavage, la membrane est exposée avec un film Hyperfilm-MP (Amersham).

Comme le montre la figure 1, pour chacune des enzymes de restriction on observe trois à quatre bandes différentes correspondant à des fragments d'ADN de taille différente qui vraisemblablement, pour certains d'entre eux, correspondent à des gènes fortement homologues à l'ICE mais différents de ce dernier puisqu'un gène unique ne peut donner qu'une ou au maximum deux bandes distinctes lors d'une hybridation avec une sonde correspondant à un seul exon.

### B - Clonage de gènes homologues à l'ICE

### a) Clonage de séquences génomiques homologues à l'ICE

Pour identifier les différents fragments d'ADN obtenus ci-dessus, les fragments de l'ADN génomique humain extrait de cellules mononucléées du sang périphérique (PBMC) puis digéré par l'enzyme HindIII (Boehringer Mannheim) ont été séparés par électrophorèse préparative en gel d'agarose 1,5 %, 1x TAE, selon les conditions décrites par Maniatis et al. (déjà cité). Le gel a été découpé en 20 fractions dans la zone correspondant aux poids moléculaires inférieurs à 2,3kb puis une amplification par PCR a été effectuée sur l'ADN élué de chacune des fractions à l'aide des oligonucléotides ICE 6.5 (SEQ ID N° 3) et ICE 6.3 (SEQ ID N° 4) décrits ci-dessus, en utilisant les conditions d'amplification suivantes : 94°C, 1 mn ; 55°C, 1 mn ; 72°C, 1 mn ; 30 cycles ; polymérase BioTaq.

Parmi les 20 fractions ci-dessus, huit fractions ayant donné la meilleure amplification par PCR ont été retenues. Le matériel amplifié a été cloné grâce à l'enzyme T4-DNA ligase dans le vecteur pCRII selon les instructions du fournisseur avec la trousse TA Cloning kit (Invitrogen) et séquence par la technique de Sanger avec l'enzyme Sequenase en utilisant le matériel d'électrophorèse Macrophor (Pharmacia System). Les séquences déterminées ont été analysées au moyen des logiciels GCG (Devereux et al. Nucleic Acids Research 12, 387-395 (1984)).

Parmi les séquences nucléotidiques obtenues, nous avons identifié une séquence appelée T2 qui présente 92 % d'identité en nucléotides avec la séquence de l'exon 6 de ICE.

La séquence nucléotidique T2 présente une phase de lecture ouverte sur l'ensemble de l'exon 6 (SEQ ID N° 5) représenté à la figure 2, qui conduit à chercher à identifier des ARN messagers codant pour une protéine T2 et à cloner l'ADNc correspondant à T2.

### b) Clonage d'ADNc homologue à l'ICE

Les ARN totaux ont été extraits et purifiés à l'aide d'une trousse RNA^{™} (Bioprobe) à partir, soit de monocytes activés par le LPS pendant 18 heures, soit de placenta, soit de polynucléaires isolés du sang périphérique. Chaque ADNc correspondant a été synthétisé à l'aide d'un oligonucléotide poly-dT et de l'enzyme transcriptase réverse en utilisant la trousse GeneAmp RNA PCR Kit (Perkin Elmer) selon les instructions du fournisseur puis amplifié par PCR en utilisant les deux oligonucléotides suivants :

| | | |
|---|---|---|
| T2.A : | CTACAGAGCTGGAGGCATTTGCT | (SEQ ID N° 6) |

choisi dans la séquence codante de l'exon 6 de T2, de façon à amplifier spécifiquement une séquence de type T2 mais non une séquence ICE, et

| | | |
|---|---|---|
| ICE45.3 | TTAATGTCCTGGGAAGAGGTAGAA | (SEQ ID N° 7) |

choisi dans l'extrémité 3' de la région codante de l'ADNc de l'ICE (brin complémentaire).

Un fragment de 600 paires de bases environ a été obtenu respectivement à partir de chacune des 3 préparations d'ARN en utilisant les conditions d'amplification suivantes : 94°C, 30 s ; 60°C, 30 s ; 72°C, 30 s ; 30 cycles avec la trousse GeneAmp RNA PCR Kit (Perkin Elmer). Le fragment a été cloné en utilisant la trousse TA Cloning kit (Invitrogen) et séquencé comme indiqué ci-dessus. La séquence nucléotidique ainsi déterminée ne correspond pas à un ADNc T2 attendu mais à un nouvel ADNc que nous avons nommé Tx.

### C - Identification de l'ADNc Tx

### a) Détermination de la séquence consensus de l'ADNc Tx

Les séquences nucléotidiques des extrémités 5' et 3' de l'ADNc de Tx ont été obtenues par la technique de PCR ancrée à partir d'un ADNc de placenta.

L'extrémité 5' de l'ADNc de Tx été amplifiée en utilisant la trousse 5'-Race-Ready cDNA (Human Quick-Clone cDNA) (Clontech) et les oligonucléotides d'amplification suivants:

| | | |
|---|---|---|
| TxPCR5A : | GAGGCAGTTG CGGTTGTTGA A | (SEQ ID N° 8) |
| TxPCR5B : | CTCTGACCCA CAGTTCCCCA C | (SEQ ID N° 9) |

L'extrémité 3' de l'ADNc Tx a été amplifiée en utilisant le kit 3' RACE System (Gibco-BRL), et les oligonucléotides d'amplification suivants :

| | | |
|---|---|---|
| TxA : | AACTGTGCAT GATGAGA | (SEQ ID N° 10) |
| TxB : | AGATGCTGTG TACAAGACC | (SEQ ID N° 11) |

Ces deux paires d'amorces respectives ont été définies à partir de la séquence partielle de Tx obtenue ci-dessus.

Les fragments d'amplification obtenus ont ensuite été clonés en utilisant la trousse TA Cloning kit (Invitrogen) et séquencés comme indiqué ci-dessus.

Les séquences nucléotidiques ont été confirmées grâce à l'utilisation des oligonucléotides TxA (SEQ ID N° 10) et TxB (SEQ ID N° 11) ci-dessus et les oligonucléotides suivants :

| | | |
|---|---|---|
| TxC : | GCCTGGACAA TGATGAC | (SEQ ID N° 12) |
| TxD : | TGATGAAGAT AGAGCCC | (SEQ ID N° 13) |
| Tx1 : | CGGGTCATGG CAGACTC | (SEQ ID N° 14) |
| Tx2 : | GTTTGAAGAA GCATTTG | (SEQ ID N° 15) |
| Tx3 : | CCTGAGTCAG GAGAATC | (SEQ ID N° 16) |
| Tx4 : | AGTCTCAGGA ATTCTTC | (SEQ ID N° 17) |
| Tx5 : | AGCTGACTTT GACATCA | (SEQ ID N° 18) |
| Tx6 : | GCGCTGACTC CATATCC | (SEQ ID N° 19) |

qui ont été choisis dans la séquence codante de Tx (brin codant ou brin complémentaire).

La compilation de l'ensemble des séquences obtenues donne la séquence nucléotidique consensus de l'ADNc Tx (SEQ ID N° 1) représentée à la figure 3. La séquence ainsi déterminée comporte 1291 nucléotides se terminant par une séquence de polyadénylation. Elle présente une phase de lecture ouverte débutant par une méthionine initiatrice au nucléotide 42 et se terminant par un codon de terminaison au nucléotide 1172. Il en résulte une phase de lecture ouverte de 1131 nucléotides codant pour une protéine de 377 acides aminés.

### b - Clonage de la région codante de l'ADNc Tx :

La région codante de l'ADNc Tx (SEQ ID N° 1) a été amplifiée par RT-PCR à partir d'ARN total soit de monocytes, soit de polynucléaires ou soit de placenta en utilisant les oligonucléotides suivants :

| | |
|---|---|
| TxP5 : | CGCGGATCCACCATGGCAGAAGGCAACCACAGA (SEQ ID N° 20) |
| TxP3 : | GGCTCTAGACTCGAGTTATCAATTGCCAGGAAAGAGGTA (SEQ ID N° 21) |

Ces amorces d'amplification ont été choisies d'après la séquence consensus ADNc Tx précédemment déterminée (ou le brin complémentaire) et synthétisées en ajoutant des sites de clonage respectivement BamHl et Nco1 pour l'oligonucléotide TxP5 et Xba1 et Xhol pour l'oligonucléotide TxP3.

Le produit amplifié obtenu ayant une longueur d'environ 1150 paires de bases a été digéré par les enzymes BamHl et Xbal (Boehringer Mannheim) et clone, en utilisant la trousse de ligation Amersham, dans le vecteur pcDNAI/Amp (Invitrogen) préalablement digéré par les mêmes enzymes de restriction BamH1 et Xba1. Le produit cloné a été entièrement séquencé sur les deux brins, au moyen des oligonucléotides TxA, TxB, TxC, TxD, Tx1, Tx2, Tx3, Tx4, Tx5 et Tx6 ci-dessus.

Pour chaque ARN de départ utilisé, la séquence nucléotidique obtenue est identique à la séquence codante de la séquence consensus ADNc Tx (SEQ ID N° 1) ci-dessus. On n'observe donc pas de différence à partir des différents tissus utilisés provenant d'individus différents.

La région codante de l'ADNc Tx code pour la protéine Tx dont la séquence déduite en acides aminés (SEQ ID N° 2) est montrée à la figure 3. La séquence de la protéine Tx comprend 377 acides aminés ayant un poids moléculaire calculé de 43,26 kDa.
L'homologie de la séquence de la protéine Tx avec le précurseur ICE p45 est au maximum de 52 % d'identité en acides aminés en introduisant des discontinuités dans l'alignement des séquences.

Un échantillon de E. coli XL-1 blue contenant la région codante de l'ADNc Tx (SEQ ID N° 1) dans le vecteur pcDNAI/Amp (cDNA Tx/pcDNAI 13/07/94) a été déposé à la CNCM le 29 juillet 1994 sous le numéro I-1462.

### c - Expression de l'ARNm de Tx dans différents tissus humains :

L'expression de l'ARNm codant pour la protéine Tx a été étudiée dans huit tissus différents par Northern Blot en utilisant une sonde d'ADN correspondant à la partie de Tx s'alignant avec l'exon 6 de l'ICE ("Tx exon 6"). Cette sonde correspond aux nucléotides 595 à 811 de la séquence SEQ ID N° 1.

La sonde "Tx exon 6" a été préparée en amplifiant cette séquence à partir du plasmide contenant l'ADNc Tx en utilisant comme amorces les oligonucléotides
T2.A (SEQ ID N° 6) et TxC (SEQ ID N° 12) ci-dessus.

Cette sonde a été marquée au ³²P par la méthode d'amorçage aléatoire "random priming" avec la trousse Oligolabelling kit (Pharmacia BioTech) puis utilisée pour détecter par hybridation des ARNm Tx sur une membrane contenant 2 µg d'ARN polyA+ respectivement de différents tissus humains sur une membrane Multiple Tissue Northern Blot II (Clontech) selon les conditions d'hybridation données par le fournisseur.

Comme le montre la figure 4, un signal d'ARNm est détecté dans la plupart des tissus testés avec des intensités variables. Les leucocytes du sang périphérique (H) donnent le signal le plus fort. La rate (A), l'intestin grèle (F), le thymus (B) et l'ovaire (E) donnent des signaux intermédiaires. La prostate (C) et le colon (G) donnent un signal très faible et enfin aucun signal n'est détecté dans l'ARNm de testicule (D).

L'ARNm codant pour la protéine Tx est donc exprimé dans de nombreux tissus et tout particulièrement dans les cellules sanguines.

### Exemple 2 : Etude de la fonction de la protéine Tx

### A - clivage de la pre-IL-1β

La capacité de la protéine Tx à cliver éventuellement le précurseur de l'IL-1β humaine a été testée dans un système de transfection en cellules eucaryotes avec l'un ou l'autre des vecteurs d'expression pcDNAI/Amp et pcDL-SRα296.

La région codante de l'ADNc Tx (SEQ ID N° 1) a d'abord été clonée aux sites BamHl et Xbal du vecteur d'expression eucaryote pcDNAI/Amp (Invitrogen). Après digestion avec les enzymes BamHl et Xbal, un insert de 1150 paires de bases environ a été isolé puis purifié. Les sites de restriction des extrémités ont été remplies à l'aide de la T4 DNA Polymérase (Boehringer Mannheim). L'ADNc obtenu a été sous-cloné à l'aide d'une trousse de ligation (Amersham) dans le vecteur pcDL-SRα296 (Takebe et al., Molecular and Cellular Biology, Vol 8, 466, 1988) ouvert par l'enzyme Xbal puis dont les extrémités ont été remplies par la T4 DNA Polymérase. Après purification en utilisant la trousse plasmid maxi kit (QIAGEN), des préparations d'ADN plasmidique Tx dans les deux vecteurs ont été obtenues.

Les cellules eucaryotes utilisées pour la transfection sont une lignée cellulaire Cos-1 qui exprime constitutivement la pIL-1β et qui a été obtenue par transfection d'un plasmide contenant le gène de la pIL-1β humaine. La synthèse de pIL-1β est maintenue dans cette lignée en cultivant les cellules en présence de 0,5 mg/ml de sulfate de G-418 dans le milieu de culture DMEM, SVF 10 %, glutamine, P/S, pyruvate, HEPES.

3x10⁶ cellules Cos-1 sont incubées à 37°C en atmosphère humide à 5 % de CO₂ dans des boites de Pétri et transfectées avec 15 µg d'ADN plasmidique préalablement mélangé à 200 µl de DEAE-Dextran et dilué avec 4 ml de PBS avant d'être ajouté dans les boites. Après incubation des cellules à 37°C pendant 30 minutes et addition de 8 ml d'une solution de chloroquine 80 µM en DMEM sans sérum, les cellules sont incubées pendant 2,5 heures. La solution surnageante est ensuite aspirée et les cellules sont traitées pendant deux minutes avec du DMSO à 10 % dans du DMEM sans sérum. Après lavage avec le milieu sans sérum, 10 ml de milieu de culture complet ci-dessus sont ajoutés. Après incubation, le surnageant des cellules transfectées est récolté à différents temps compris entre 16 et 45 heures.

L'IL-1β mature présente dans les surnageants est mesurée par un test ELISA IL1-β (R&D Systems) qui permet la détection spécifique de l'IL-1β mature.

La transfection a été réalisée avec la région codante de l'ADNc Tx inséré dans l'un ou l'autre des deux vecteurs ci-dessus en comparaison avec des transfections comprenant respectivement la région codante de l'ADNc de ICE p45 ou de ICE p30 ainsi qu'une transfection témoin avec le vecteur seul correspondant ne contenant pas de plasmide.

Comme le montre la figure 5, la transfection de l'ADNc de l'ICE p45 (colonne 2) ou p30 (colonne 7) confère aux cellules la capacité à sécréter de l'IL-1β mature. Par contre, lorsque l'ADNc Tx est transfecté dans les mêmes conditions (colonnes 3 et 6), on n'observe pas d'IL-1β sécrétée comme pour les transfections contrôles (colonnes 1, 4, 5). Des résultats semblables sont obtenus avec les deux vecteurs d'expression quelque soit le temps d'incubation (16 h : fig. 5A ; 24 h : fig. 5B ; 29 h et jusqu'à 44 h) après la transfection.

La protéine Tx ne possède pas la propriété de convertase de l'IL-1β.

### B - Activité protéase de la protéine Tx : clivage du précurseur 30 kDa de ICE

La capacité de la protéine Tx à cliver éventuellement le précurseur 30 kDa de l'ICE (ICE p30) a été testée dans un système de co-transfection dans des cellules eucaryotes en introduisant simultanément dans des cellules Cos-l un vecteur contenant la région codante de l'ADNc Tx (SEQ ID N° 1) et un vecteur contenant un ADN codant pour une protéine ICE modifiée, chaque ADN étant respectivement inséré dans le vecteur d'expression pcDL-SRα296 ci-dessus.

La protéine ICE a été doublement modifiée : D'une part, pour permettre une détection spécifique de la protéine ICE en présence de la protéine Tx, un petit peptide T7 a été fusionné à l'extrémité N terminale de la protéine ICE p30. La protéine ainsi marquée ou ses produits de maturation est détectable par Western Blotting avec un anticorps monoclonal spécifique du peptide T7 (Tsai et al., Proc. Natl. Acad. Sci., 89, 8864, 1992). D'autre part pour exprimer l'enzyme sous une forme incapable d'induire sa propre maturation, un mutant de l'ICE p30, dont la cystéine Cys 285 du site actif a été remplacée par une sérine, a été utilisé (Wilson, K.P. et al. Nature, 370, 28 July 1994, 270-275). Cette enzyme est alors inactive et incapable d'induire sa propre maturation ou de cliver la pIL-1β. Le mutant a été préparé par mutagénèse dirigée à l'aide d'oligonucléotides appropriés et de la trousse de mutagénèse Transformer^{™} site-directed mutagenesis kit (Clontech). La séquence obtenue a été entièrement vérifiée. On a ainsi obtenu l'ICE p30 marquée et mutée désignée T7-ICEp30C285S.

Les cellules Cos-1 ont été transfectées soit par le vecteur pcDL-SRα296 contenant T7-ICEp30C285S, soit par le vecteur pcDL-SRα296 contenant Tx ou co-transfectées par les deux vecteurs, en suivant les conditions opératoires ci-dessus. Après 22 heures de culture, les cellules ont été récoltées, lavées et lysées dans un tampon NaCl 10mM, Hepes 10 mM, pH 7.4, EDTA 1 mM, NaF 50-mM, Triton X-100 0,2 %, leupeptine 1 µg/ml, aprotinine 20 u/*µ*l et PMSF 1 mM. Le lysat cellulaire a été centrifugé à 400 g et 4°C puis le surnageant a été soumis à une électrophorèse sur un gel à 16 % de polyacrylamide en SDS-PAGE. Les protéines du gel ont ensuite été transférées sur une membrane de nitrocellulose et incubées avec l'anticorps monoclonal de souris anti-T7 (Novagen) pendant 2 heures à température ambiante. La membrane a été lavée puis incubée 1 heure à température ambiante avec un anticorps de chèvre anti-immunoglobuline de souris conjugué à la phosphatase alcaline. Les anticorps fixés à la membrane sont ensuite révélés par le substrat de la phosphatase alcaline (Promega).

Des co-transfections ont été réalisées de la même façon avec le vecteur pcDL-SRα296 contenant T7-ICEp30C285S et le vecteur pcDL-SRα296 contenant soit ICE p30, soit ICE p45 à la place de Tx.

Comme le montre la figure 6, lorsque la protéine ICE p30 mutée (T7-ICEp30C285S) est exprimée seule dans les cellules Cos transfectées, la forme T7-p30 de l'enzyme (PM apparent 35 kDa) peut être détectée (ligne B).
L'absence de bande correspondant à la forme p20 montre que l'enzyme mutée est incapable de se cliver. Lorsque les cellules sont co-transfectées avec le vecteur contenant l'enzyme ICE p30 (ligne C) ou p45 (ligne D), on observe une bande à un PM apparent de 26 kDa correspondant au produit de clivage T7-p20 par l'enzyme active. Lorsque les cellules sont co-transfectées avec le vecteur contenant Tx (ligne E), on observe également l'apparition d'une bande majoritaire à un PM apparent de 26 kDa accompagnée de deux bandes mineures à environ 31 kDa et 28 kDa. Les différentes bandes correspondent aux produits de clivage de la forme p30 de l'ICE par la protéine Tx exprimée dans les cellules.

Ces résultats montrent que la protéine Tx, d'une part est exprimée dans les cellules Cos tranfectées, d'autre part possède une activité protéase. De plus, la protéine Tx est capable de cliver le précurseur 30 kDa de l'ICE et peut ainsi contribuer à la maturation in vivo du pro-enzyme de l'ICE et à la génération de l'enzyme sous forme active.

### C - Induction de l'apoptose par la protéine Tx

La capacité de la protéine Tx à induire l'apoptose a été testée par transfection dans des cellules Cos et par examen morphologique des cellules cultivées.

La transfection de l'ICE dans différents types cellulaires entrainant la mort de ces cellules par apoptose a été décrite (Miura et al. déjà cité).

La transfection de cellules Cos-1 par le vecteur pcDL-SRα296 contenant la région codante de l'ADNc Tx (SEQ ID N° 1) ainsi que la transfection avec le vecteur pcDL-SRα296 contenant ICE p45 ont été réalisées comme décrit ci-dessus. La morphologie des cellules a été observée après une incubation de 22 heures.

Comme le montre la figure 7, on observe l'apparition de cellules rondes qui se détachent du support et dont l'aspect morphologique est caractéristique des cellules en apoptose dans les cultures de cellules Cos transfectées avec l'ADNc de l'ICE (7D). Le même changement de morphologie est observé dans les cultures de cellules Cos transfectées avec l'ADNc Tx (7C).

Des résultats morphologiques identiques ont été obtenus lorsqu'on utilise le vecteur pcDNAI/Amp ci-dessus pour exprimer ICE et Tx dans les cellules Cos-1 transfectées.

Ces résultats ont été confirmés par l'observation de l'ADN isolé à partir des cellules Cos-1 transfectées et incubées 40 heures après la transfection. L'ADN des cellules a été préparé avec la trousse microTurboGen (Invitrogen), migré sur gel d'agarose 1,5 % et coloré par le BET.

Comme le montre la figure 8, l'ADN des cellules transfectées par ICE p45 (ligne B) et par Tx (ligne C) présente l'aspect caractéristique "en échelle" de cellules en apoptose.

Des cellules transfectées sans ADN (7A et 8A) ou avec le vecteur ne contenant pas d'ADNc (7B et 8D) ne présentent ni la morphologie ni l'ADN de cellules en apoptose.

Ces résultats montrent que la protéine Tx est impliquée dans l'induction de l'apoptose.

### Exemple 3 : Identification de la séquence Ty

### A - Clonage de gènes homologues à Tx

L'ADN génomique humain extrait de cellules mononuclées du sang périphérique a été digéré par l'enzyme Hind III (Boehringer Mannheim), puis les fragments ont été séparés par électrophorèse préparative en gel d'agarose 1 %, TAE 1x, selon les conditions décrites par Maniatis et al., déjà cité. Le gel a été découpé en 24 fractions dans la zone du puits de dépôt correspondant aux poids moléculaires supérieurs à 1,9 kb puis une amplification par PCR a été effectuée sur l'ADN élué de chacune des fractions à l'aide des oligonucléotides T2A (SEQ ID N° 6) et TxC (SEQ ID N° 12) déjà décrits et en utilisant les conditions d'amplification suivantes : 94°C, 30 sec ; 55°C, 30 sec ; 72°C, 1 mn ; 30 cycles ; polymérase BioTaq (BioProbe).

Parmi les 24 fractions ci-dessus, 10 fractions ayant donné la meilleure amplification par PCR ont été retenues. Le matériel amplifié a été purifié sur gel d'agarose et a été cloné dans le vecteur pCRII avec la trousse TA Cloning kit (Invitrogen) et séquencé par la technique de Sanger avec l'enzyme Sequenase (Version 2.0 DNA Sequencing Kit) en utilisant le système d'électrophorèse Macrophor (Pharmacia System). Les séquences déterminées ont été analysées au moyen des logiciels GCG comme indiqué à l'exemple 1.

Nous avons identifié une séquence nucléotidique appelée Ty qui présente 94,9 % d'identité en nucléotides avec la séquence ADNc Tx (SEQ ID N° 1) et qui conduit à chercher à cloner l'ADNc correspondant à Ty.

### B - Clonage et identification de l'ADNc Ty

### a) Détermination de la séquence consensus de l'ADNc Ty

Les séquences nucléotidiques des extrémités 5' et 3' de l'ADNc de Ty ont été obtenues respectivement à partir d'ADNc de rate et de placenta humains par la technique de PCR ancrée.

L'extrémité 3' de l'ADNc de Ty a été amplifiée en utilisant le kit 3'RACE System (Gibco-BRL), et les oligonucléotides d'amplification suivants :

| | | |
|---|---|---|
| Ty 3.2 : | CATGTCTCATGGCATCCTA | (SEQ ID N° 24) et |
| Ty 3.1 : | CTGCGGAACTGCGCATAAAA | (SEQ ID N° 25). |

Ces deux amorces ont été définies à partir de la séquence partielle de l'exon 6 de la séquence Ty obtenue ci-dessus. Les fragments amplifiés ont été purifiés sur gel d'agarose, clonés dans le vecteur pCRII et séquencés comme indiqué ci-dessus. La compilation des séquences obtenues a permis de définir la partie 3' de la région codante de l'ADNc Ty ainsi que la région 3' non codante.

L'extrémité 5' de l'ADNc de Ty a été amplifiée en utilisant le kit Human Spleen 5'-RACE-Ready cDNA (Clontech) et les oligonucléotides d'amplification suivants :

| | | |
|---|---|---|
| Ty5A1 : | GGCTCTAGACTCGAGGTGCTCTTTGATGTTGACAG | (SEQ ID N° 26) |
| et | | |
| Ty5A4 : | CTTCTCCTCGTGGATCTTGC | (SEQ ID N° 27). |

Ces deux amorces ont été définies à partir de la séquence de la région 3' de l'ADNc Ty obtenue ci-dessus en ajoutant des sites de restriction Xbal et Xhol pour Ty5A1. Les fragments d'amplification ont ensuite été clonés en utilisant la trousse TA Cloning kit (Invitrogen) et séquencés comme indiqué ci-dessus.

Les séquences nucléotidiques ont été confirmées en utilisant des oligonuclotides Ty5A1 (SEQ ID N° 26) et Ty 3.1 (SEQ ID N° 25) ci- dessus et les oligonucléotides suivants:

| | | |
|---|---|---|
| Ty 2 : | AGATGTTCTTCATGGT | (SEQ ID N° 28) |
| Ty 4 : | CTTCTCAATATGGACCA | (SEQ ID N° 29) |
| Ty 5 : | CCTGGCTCTCATCATAT | (SEQ ID N° 30) |
| Ty 6 : | ATTTGCTGCCAGACCAGA | (SEQ ID N° 31) |
| Ty 7 : | GCCTGCAGAGGTGAAAAAC | (SEQ ID N° 32) |
| Ty 8 : | GCTCCATCTTCATTACGGA | (SEQ ID N° 33) |
| Ty 0 : | GATTTCTGTACCTTCCG | (SEQ ID N° 34) |
| Ty A : | TTTATGCGCAGTTCCG | (SEQ ID N° 35) |
| Ty B : | GTCATAGTGAGCCCCATT | (SEQ ID N° 36) |
| Ty C : | CTTCACGAGGACAAAGT | (SEQ ID N° 37) |
| Ty D : | TCGCAAAGAGTCTACCA | (SEQ ID N° 38) |

Ces oligonucléotides ont été choisis dans la séquence codante de Ty (brin codant ou brin complémentaire).

La compilation de l'ensemble des séquences obtenues donne la séquence nucléotidique consensus de l'ADNc Ty (SEQ ID N° 22).

### b) Clonage de la région codante de l'ADNc Ty

La région codante de l'ADNc Ty (SEQ ID N° 22) a été amplifiée par PCR à partir de l'ADNc de rate ou de placenta humains en utilisant les kits correspondant 5'-RACE-Ready cDNA (Clontech), l'oligonucléotide Ty5A1 (SEQ ID N° 26) ci-dessus et l'oligonucléotide suivant :

| | | |
|---|---|---|
| TyP5 : | CGCGGATCCAAGATGTTGGAATACCTGGGCAAA | (SEQ ID N° 39). |

Ces amorces d'amplification ont été choisies d'après la séquence consensus de l'ADNc Ty (SEQ ID N° 22) déterminée ci-dessus en ajoutant le site de clonage BamHl pour TyP5.

Le produit amplifié et purifié sur gel d'agarose ayant une longueur d'environ 1100 paires de bases a été digéré par les enzymes de restriction BamHl et Xba1 puis cloné dans le vecteur pcDNAI/Amp comme décrit à l'exemple 1 et séquencé comme indiqué ci-dessus. Le produit cloné a été entièrement séquencé sur les deux brins à l'aide des oligonucléotides SEQ ID N° 28 à SEQ ID N° 38 définis ci-dessus.

Une séquence identique à la séquence codante de la séquence consensus ADNc Ty (SEQ ID N° 22) a été obtenue. L'homologie de la séquence codante de l'ADNc Ty avec la séquence codante de l'ADNc Tx obtenu à l'exemple 1 est de 84 % d'identité en nucléotides.

La région codante de l'ADNc Ty code pour la protéine Ty ayant la séquence déduite en acides aminés SEQ ID N° 23. La séquence de la protéine comprend 364 acides aminés ayant un poids moléculaire calculé de 41,8 kDa.

L'homologie de séquence de la protéine Ty avec la protéine Tx obtenue à l'exemple 1 est de 75 % d'identité en acides aminés.

Un échantillon de E. coli XL-1 blue contenant la région codante de l'ADNc Ty (SEQ ID N' 22) dans le vecteur pcDNAI/Amp (cDNA Ty/pcDNAI/Amp 30/6/95) a été déposé à la CNCM le 5 juillet 1995 sous le n° I-1068.

### Exemple 4 : Activités biologiques de la protéine Ty

### A - Induction de l'apoptose :

La capacité de la protéine Ty à induire l'apoptose a été testée selon le mode opératoire indiqué à l'exemple 2, à l'aide de cellules Cos-1 transfectées par le vecteur pcDL-SRα296 contenant la région codante de l'ADNc Ty (SEQ ID N° 22) dénommé pcDL-TY, et dont la préparation a été réalisée en suivant les conditions décrites à l'exemple 2 pour le sous-clonage de l'ADNc Tx.

La morphologie des cellules a été observée après une incubation de 23 heures et de 43 heures et l'observation de l'ADN isolé a été effectuée après une incubation de 43 heures.

Les résultats, obtenus comparativement à des cellules transfectées par le vecteur contenant la région codante de Tx ou de ICE p45, sont identiques à ceux montrés pour la protéine Tx à la figure 7 et à la figure 8 de l'exemple 2.

Ces résultats montrent que la protéine Ty comme la protéine Tx est impliquée dans l'induction de l'apoptose.

### B - Activité protéase de la protéine Ty :

La capacité de la protéine Ty à s'autocliver de façon intermoléculaire a été testée dans un système de co-transfection dans des cellules eucaryotes, dans des conditions analogues à celles décrites pour le clivage du précurseur de l'ICE par la protéine Tx à l'exemple 2, en introduisant simultanément dans des cellules Cos-1 un vecteur contenant la région codante de l'ADNc Ty (SEQ ID N° 22) et un vecteur contenant un ADN codant pour une protéine Ty modifiée, chaque ADN étant inséré respectivement dans le vecteur d'expression pcDL-SRα296 et dans le vecteur pcDNAI/Amp décrits à l'exemple 2.

La protéine Ty a été doublement modifiée, d'une part le codon Cys 245 a été muté en un codon Serine par la méthode de PCR "chevauchante", d'autre part l'épitope tag T7 (MASMTGGQQMG) a été introduit à l'extrémité N-terminale du fragment de Ty correspondant aux résidus 68 à 364 de la séquence SEQ ID N° 23.

Les paires d'amorces suivantes ont été utilisées pour amplifier la matrice ADNc Ty :
**a)** d'une part,
   T7TY :
   CGCGGATCCACCATGGCTTCTATGACAGGAGGTCAACAAATGGGACAAAAGATCACCAGTG TAAAACC (SEQ ID N° 40)
   choisie dans la séquence codante de Ty et synthétisée en ajoutant un site de restriction BamH1 suivi de la séquence nucléotidique codant pour le tag T7 et
   TYC245SR :
   ATGTTTTTCACCTCTGGAGGCCTGGACAATGATGAC (SEQ ID N° 41)
   choisie dans la séquence codante de Ty (brin complémentaire) avec une mutation C -> G en position 17,
**b)** d'autre part,
   TYC245S:
   GTCATCATTGTCCAGGCCTCCAGAGGTGAAAAACAT (SEQ ID N° 42)
   choisie dans la séquence codante de Ty avec une mutation
   G -> C en position 20 et
   Ty5A1 (SEQ ID N° 26) ci-dessus,
   et en utilisant les conditions d'amplification suivantes : 94°C, 1 mn ; 60°C, 1 mn ; 72°C, 1 mn ; 30 cycles ; Vent polymérase (Biolabs).
Les deux produits d'amplification obtenus respectivement ont été combinés et amplifiés par PCR en utilisant les amorces T7Ty et Ty5Al et les conditions ci-dessus.

Le produit d'amplification a été digéré avec les enzymes de restriction BamHl et Xbal puis cloné dans le vecteur pcDNAI/Amp digéré au préalable par les mêmes enzymes de restriction. Le plasmide résultant est dénommé pT7TYΔ67C245S. La séquence obtenue a été entièrement vérifiée par séquençage d'ADN.

Le vecteur d'expression pcDL-SRα296 dans lequel a été sous-cloné la séquence d'ADNC Ty (SEQ ID N° 22), dénommé plasmide pcDL-TY, a été préparé comme indiqué ci-dessus.

Les cellules Cos-1 ont été soit transfectées par le vecteur pT7TYΔ67C245S, soit co-transfectées avec ce plasmide et le plasmide pcDL-TY puis cultivées pendant 23 heures ou 43 heures, lysées et analysées par électrophorèse sur gel de polyacrylamide et Western blot en utilisant l'anticorps monoclonal anti-T7 de souris, selon les conditions opératoires décrites à l'exemple 2, mais dans lesquelles l'anticorps de chèvre anti-immunoglobine de souris est conjugué à la peroxydase de raifort au lieu de la phosphatase alcaline. Les anticorps liés à la membrane sont ensuite révélés avec le système de détection Western ECL (Amersham) par autoradiographie.

Des co-transfections ont été réalisées de la même façon avec le vecteur pcDL-SRα296 seul et le vecteur pcDNAI/Amp seul et les cellules ont été cultivées pendant 23 heures.

Comme le montre la figure 9, lorsque la protéine Ty mutée est exprimée seule dans les cellules Cos transfectées, la forme T7Ty (PM apparent environ 30 kDa) peut être détectée (ligne E). L'absence de bande de PM inférieur montre que la protéine Ty mutée est incapable de s'autocliver. Lorsque les cellules sont co-transfectées avec le vecteur contenant Ty (lignes F et G), on observe l'apparition d'une bande majoritaire à un PM apparent de 20 kDa correspondant au produit de clivage de la protéine Ty mutée par la protéine Ty.

Ces résultats montrent que la protéine Ty d'une part est exprimée dans les cellules Cos transfectées, d'autre part possède une activité protéase et qu'elle est en particulier capable de s'autocliver de façon intermoléculaire.

### LISTE DE SEQUENCES

(1) INFORMATIONS GENERALES:
   (i) DEPOSANT:
      (A) NOM: ROUSSEL UCLAF
      (B) RUE: 102, Route de Noisy
      (C) VILLE: ROMAINVILLE
      (E) PAYS: FRANCE
      (F) CODE POSTAL: 93230
      (G) TELEPHONE: 49.91.49.91
      (H) TELECOPIE: 49.91.46.10
   (ii) TITRE DE L' INVENTION: Sequence d'ADN codant pour une proteine humaine Tx apparentee a l'enzyme de conversion de l'interleukine-lbeta, proteine Tx, procede de production, compositions pharmaceutiques et leurs applications.
   (iii) NOMBRE DE SEQUENCES: 42
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB) + Corrections sous WORDPERFECT 5.1 pour SEQ ID NO 22 (ix)
   (vi) DONNEES DE LA DEMANDE ANTERIEURE:
      (A) NUMERO DE LA DEMANDE: FR 9409567
      (B) DATE DE DEPOT: 02-AUG-1994
(2) INFORMATIONS POUR LA SEQ ID NO: 1:
   (i) CARACTERISTIQUES DE LA SEQUENCE:
      (A) LONGUEUR: 1291 paires de bases
      (B) TYPE: nucléotide
      (C) NOMBRE DE BRINS: simple
      (D) CONFIGURATION: linéaire
   (ii) TYPE DE MOLECULE: ADNc
   (vi) ORIGINE:
      (A) ORGANISME: Homo sapiens
   (ix) CARACTERISTIQUE:
      (A) NOM/CLE: CDS
      (B) EMPLACEMENT:42..1172
   (xi) DESCRIPTION DE LA SEQUENCE: SEQ ID NO: 1:
   GCTCTTTCCA ACGCTGTAAA AAAGGACAGA GGCTGTTCCC T ATG GCA GAA GGC       53
                                                 Met Ala Glu Gly
                                                  1
   AAC CAC AGA AAA AAG CCA CTT AAG GTG TTG GAA TCC CTG GGC AAA GAT     101
   Asn His Arg Lys Lys Pro Leu Lys Val Leu Glu Ser Leu Gly Lys Asp
    5                  10                  15                  20
   TTC CTC ACT GGT GTT TTG GAT AAC TTG GTG GAA CAA AAT GTA CTG AAC     149
   Phe Leu Thr Gly Val Leu Asp Asn Leu Val Glu Gln Asn Val Leu Asn
                   25                  30                  35
   TGG AAG GAA GAG GAA AAA AAG AAA TAT TAC GAT GCT AAA ACT GAA GAC     197
   Trp Lys Glu Glu Glu Lys Lys Lys Tyr Tyr Asp Ala Lys Thr Glu Asp
               40                  45                  50
   AAA GTT CGG GTC ATG GCA GAC TCT ATG CAA GAG AAG CAA CGT ATG GCA     245
   Lys Val Arg Val Met Ala Asp Ser Met Gln Glu Lys Gln Arg Met Ala
           55                  60                  65
   GGA CAA ATG CTT CTT CAA ACC TTT TTT AAC ATA GAC CAA ATA TCC CCC     293
   Gly Gln Met Leu Leu Gln Thr Phe Phe Asn Ile Asp Gln Ile Ser Pro
       70                  75                  80
   AAT AAA AAA GCT CAT CCG AAT ATG GAG GCT GGA CCA CCT GAG TCA GGA     341
   Asn Lys Lys Ala His Pro Asn Met Glu Ala Gly Pro Pro Glu Ser Gly
   85                  90                  95                  100
   GAA TCT ACA GAT GCC CTC AAG CTT TGT CCT CAT GAA GAA TTC CTG AGA     389
   Glu Ser Thr Asp Ala Leu Lys Leu Cys Pro His Glu Glu Phe Leu Arg
                   105                 110                 115
   CTA TGT AAA GAA AGA GCT GAA GAG ATC TAT CCA ATA AAG GAG AGA AAC     437
   Leu Cys Lys Glu Arg Ala Glu Glu Ile Tyr Pro Ile Lys Glu Arg Asn
               120                 125                 130
   AAC CGC ACA CGC CTG GCT CTC ATC ATA TGC AAT ACA GAG TTT GAC CAT     485
   Asn Arg Thr Arg Leu Ala Leu Ile Ile Cys Asn Thr Glu Phe Asp His
           135                 140                 145
   CTG CCT CCG AGG AAT GGA GCT GAC TTT GAC ATC ACA GGG ATG AAG GAG        533
   Leu Pro Pro Arg Asn Gly Ala Asp Phe Asp Ile Thr Gly Met Lys Glu
       150                 155                 160
   CTA CTT GAG GGT CTG GAC TAT AGT GTA GAT GTA GAA GAG AAT CTG ACA        581
   Leu Leu Glu Gly Leu Asp Tyr Ser Val Asp Val Glu Glu Asn Leu Thr
   165                 170                 175                 180
   GCC AGG GAT ATG GAG TCA GCG CTG AGG GCA TTT GCT ACC AGA CCA GAG        629
   Ala Arg Asp Met Glu Ser Ala Leu Arg Ala Phe Ala Thr Arg Pro Glu
                   185                 190                 195
   CAC AAG TCC TCT GAC AGC ACA TTC TTG GTA CTC ATG TCT CAT GGC ATC        677
   His Lys Ser Ser Asp Ser Thr Phe Leu Val Leu Met Ser His Gly Ile
               200                 205                 210
   CTG GAG GGA ATC TGC GGA ACT GTG CAT GAT GAG AAA AAA CCA GAT GTG        725
   Leu Glu Gly Ile Cys Gly Thr Val His Asp Glu Lys Lys Pro Asp Val
           215                 220                 225
   CTG CTT TAT GAC ACC ATC TTC CAG ATA TTC AAC AAC CGC AAC TGC CTC        773
   Leu Leu Tyr Asp Thr Ile Phe Gln Ile Phe Asn Asn Arg Asn Cys Leu
       230                 235                 240
   AGT CTG AAG GAC AAA CCC AAG GTC ATC ATT GTC CAG GCC TGC AGA GGT        821
   Ser Leu Lys Asp Lys Pro Lys Val Ile Ile Val Gln Ala Cys Arg Gly
   245                 250                 255                 260
   GCA AAC CGT GGG GAA CTG TGG GTC AGA GAC TCT CCA GCA TCC TTG GAA        869
   Ala Asn Arg Gly Glu Leu Trp Val Arg Asp Ser Pro Ala Ser Leu Glu
                   265                 270                 275
   GTG GCC TCT TCA CAG TCA TCT GAG AAC CTG GAG GAA GAT GCT GTT TAC        917
   Val Ala Ser Ser Gln Ser Ser Glu Asn Leu Glu Glu Asp Ala Val Tyr
               280                 285                 290
   AAG ACC CAC GTG GAG AAG GAC TTC ATT GCT TTC TGC TCT TCA ACG CCA        965
   Lys Thr His Val Glu Lys Asp Phe Ile Ala Phe Cys Ser Ser Thr Pro
           295                 300                 305
   CAC AAC GTG TCC TGG AGA GAC AGC ACA ATG GGC TCT ATC TTC ATC ACA       1013
   His Asn Val Ser Trp Arg Asp Ser Thr Met Gly Ser Ile Phe Ile Thr
       310                 315                 320
   CAA CTC ATC ACA TGC TTC CAG AAA TAT TCT TGG TGC TGC CAC CTA GAG       1061
   Gln Leu Ile Thr Cys Phe Gln Lys Tyr Ser Trp Cys Cys His Leu Glu
   325                 330                 335                 340
   GAA GTA TTT CGG AAG GTA CAG CAA TCA TTT GAA ACT CCA AGG GCC AAA       1109
   Glu Val Phe Arg Lys Val Gln Gln Ser Phe Glu Thr Pro Arg Ala Lys
                   345                 350                 355
   GCT CAA ATG CCC ACC ATA GAA CGA CTG TCC ATG ACA AGA TAT TTC TAC       1157
   Ala Gln Met Pro Thr Ile Glu Arg Leu Ser Met Thr Arg Tyr Phe Tyr
               360                 365                 370
   CTC TTT CCT GGC AAT TGAAAATGGA AGCCACAAGC AGCCCAGCCC TCCTTAATCA       1212
   Leu Phe Pro Gly Asn
           375
   ACTTCAAGGA GCACCTTCAT TAGTACAGCT TGCATATTTA ACATTTTGTA TTTCAATAAA    1272
   AGTGAAGACA AAAAAAAAA                                                 1291
(2) INFORMATIONS POUR LA SEQ ID NO: 2:
   (i) CARACTERISTIQUES DE LA SEQUENCE:
      (A) LONGUEUR: 377 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Ala Glu Gly Asn His Arg Lys Lys Pro Leu Lys Val Leu Glu Ser
   1 5 10 15
   Leu Gly Lys Asp Phe Leu Thr Gly Val Leu Asp Asn Leu Val Glu Gln
   20 25 30
   Asn Val Leu Asn Trp Lys Glu Glu Glu Lys Lys Lys Tyr Tyr Asp Ala
   35 40 45
   Lys Thr Glu Asp Lys Val Arg Val Met Ala Asp Ser Met Gln Glu Lys
   50 55 60
   Gln Arg Met Ala Gly Gln Met Leu Leu Gln Thr Phe Phe Asn Ile Asp
   65 70 75 80
   Gln Ile Ser Pro Asn Lys Lys Ala His Pro Asn Met Glu Ala Gly Pro
   85 90 95
   Pro Glu Ser Gly Glu Ser Thr Asp Ala Leu Lys Leu Cys Pro His Glu
   100 105 110
   Glu Phe Leu Arg Leu Cys Lys Glu Arg Ala Glu Glu Ile Tyr Pro Ile
   115 120 125
   Lys Glu Arg Asn Asn Arg Thr Arg Leu Ala Leu Ile Ile Cys Asn Thr
   130 135 140
   Glu Phe Asp His Leu Pro Pro Arg Asn Gly Ala Asp Phe Asp Ile Thr
   145 150 155 160
   Gly Met Lys Glu Leu Leu Glu Gly Leu Asp Tyr Ser Val Asp Val Glu
   165 170 175
   Glu Asn Leu Thr Ala Arg Asp Met Glu Ser Ala Leu Arg Ala Phe Ala
   180 185 190
   Thr Arg Pro Glu His Lys Ser Ser Asp Ser Thr Phe Leu Val Leu Met
   195 200 205
   Ser His Gly Ile Leu Glu Gly Ile Cys Gly Thr Val His Asp Glu Lys
   210 215 220
   Lys Pro Asp Val Leu Leu Tyr Asp Thr Ile Phe Gln Ile Phe Asn Asn
   225 230 235 240
   Arg Asn Cys Leu Ser Leu Lys Asp Lys Pro Lys Val Ile Ile Val Gln
   245 250 255
   Ala Cys Arg Gly Ala Asn Arg Gly Glu Leu Trp Val Arg Asp Ser Pro
   260 265 270
   Ala Ser Leu Glu Val Ala Ser Ser Gln Ser Ser Glu Asn Leu Glu Glu
   275 280 285
   Asp Ala Val Tyr Lys Thr His Val Glu Lys Asp Phe Ile Ala Phe Cys
   290 295 300
   Ser Ser Thr Pro His Asn Val Ser Trp Arg Asp Ser Thr Met Gly Ser
   305 310 315 320
   Ile Phe Ile Thr Gln Leu Ile Thr Cys Phe Gln Lys Tyr Ser Trp Cys
                   325                 330                 335
   Cys His Leu Glu Glu Val Phe Arg Lys Val Gln Gln Ser Phe Glu Thr
               340                 345                 350
   Pro Arg Ala Lys Ala Gln Met Pro Thr Ile Glu Arg Leu Ser Met Thr
           355                 360                 365
   Arg Tyr Phe Tyr Leu Phe Pro Gly Asn
       370                 375
(2) INFORMATIONS POUR LA SEQ ID NO: 3:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (x) INFORMATION DE LA PUBLICATION:
      (A) AUTEURS: Thornberry, Nancy A.
         Bull, Herbert G.
         Calaycay, Jimmy R.
         Chapman, Kevin T.
         Howard, Andrew D.
         Kostura, Matthew J.
         Miller, Douglas K.
         Molineaux, Susan M.
         Weidner, Jeffrey R.
         Aunins, John
      (B) TITRE: A novel heterodimeric cysteine protease is required for interleukin-lbeta processing in monocytes
      (C) REVUE: Nature
      (D) VOLUME: 356
      (F) PAGES: 768-774
      (G) DATE: 30-APRIL-1992
      (K) RESIDUES PERTINENTS DANS LA SEQ ID NO: 3: DE 1 JUSQU'AU 20
   (xi) DESCRIPTION DE LA SEQUENCE: SEQ ID NO: 3:
      ACATGACTAC AGAGCTGGAG 20
(2) INFORMATIONS POUR LA SEQ ID NO: 4:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..20)
   (x) INFORMATION DE LA PUBLICATION:
      (A) AUTEURS: Thornberry, Nancy A.
         Bull, Herbert G.
         Calaycay, Jimmy R.
         Chapman, Kevin T.
         Howard, Andrew D.
         Kostura, Matthew J.
         Miller, Douglas K.
         Molineaux, Susan M.
         Weidner, Jeffrey R.
         Aunins, John
      (B) TITRE: A novel heterodimeric cysteine protease is required for interleukin-1beta processing in monocytes
      (C) REVUE: Nature
      (D) VOLUME: 356
      (F) PAGES: 768-774
      (G) DATE: 30-APRIL-1992
   (xi) DESCRIPTION DE LA SEQUENCE: SEQ ID NO: 4:
      CACCACGGCA GGCCTGGATG 20
(2) INFORMATIONS POUR LA SEQ ID NO: 5:
   (i) CARACTERISTIQUES DE LA SEQUENCE:
      (A) LONGUEUR: 235 paires de bases
      (B) TYPE: nucléotide
      (C) NOMBRE DE BRINS: double
      (D) CONFIGURATION: linéaire
   (ii) TYPE DE MOLECULE: ADN (génomique)
   (vi) ORIGINE:
      (A) ORGANISME: Homo sapiens
   (xi) DESCRIPTION DE LA SEQUENCE: SEQ ID NO: 5:
   GAAATGACTA CAGAGCTGGA GGCATTTGCT CACCGCCCAG AGCACAAGAC CTCTGACAGC    60
   ACCTTCCCGG TGTTCTTGTC TCATGGTGTT CGGGAAGGCA TTTGTGGGAA GAAATACTCT   120
   GAACAAGTCC CTGATATATT ACAATTCAAT GAAATATTTA AAATGTTGAA TAGCAAGAAC   180
   TGCCCAAGTT TGAAGGACAA ACCCAAGGTG ATCATCTTCG AGGCCTGCTG TGGTG        235
(2) INFORMATIONS POUR LA SEQ ID NO: 6:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..23
      (D) AUTRES INFORMATIONS:/note= "SEQ ID NO 5 DE 8 A 30"
   (xi) DESCRIPTION DE LA SEQUENCE: SEQ ID NO: 6:
      CTACAGAGCT GGAGGCATTT GCT 23
(2) INFORMATIONS POUR LA SEQ ID NO: 7:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..24)
   (x) INFORMATION DE LA PUBLICATION:
      (A) AUTEURS: Thornberry, Nancy A.
         Bull, Herbert G.
         Calaycay, Jimmy R.
         Chapman, Kevin T.
         Howard, Andrew D.
         Kostura, Matthew J.
         Miller, Douglas K.
         Molineaux, Susan M.
         Weidner, Jeffrey R.
         Aunins, John
      (B) TITRE: A novel heterodimeric cysteine protease is required for interleukin-1beta processing in monocytes
      (C) REVUE: Nature
      (D) VOLUME: 356
      (F) PAGES: 768-774
      (G) DATE: 30-APRIL-1992
   (xi) DESCRIPTION DE LA SEQUENCE: SEQ ID NO: 7:
      TTAATGTCCT GGGAAGAGGT AGAA 24
(2) INFORMATIONS POUR LA SEQ ID NO: 8:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..21)
      (D) AUTRES INFORMATIONS:/note= "SEQ ID NO 1 COMPLEMENTAIRE DE 753 A 773"
   (xi) DESCRIPTION DE LA SEQUENCE: SEQ ID NO: 8:
      GAGGCAGTTG CGGTTGTTGA A 21
(2) INFORMATIONS POUR LA SEQ ID NO: 9:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..21)
      (D) AUTRES INFORMATIONS:/note= "SEQ ID NO 1 COMPLEMENTAIRE DE 829 A 849"
   (xi) DESCRIPTION DE LA SEQUENCE: SEQ ID NO: 9:
      CTCTGACCCA CAGTTCCCCA C 21
(2) INFORMATIONS POUR LA SEQ ID NO: 10:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..17
      (D) AUTRES INFORMATIONS:/note= "SEQ ID NO 1 DE 695 A 711"
   (xi) DESCRIPTION DE LA SEQUENCE: SEQ ID NO: 10:
      AACTGTGCAT GATGAGA 17
(2) INFORMATIONS POUR LA SEQ ID NO: 11:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..9
      (D) AUTRES INFORMATIONS:/note= "SEQ ID NO 1 DE 905 A 913"
   (ix) CARACTERISTIQUE:
      (A) NOM/CLE: misc_feature
      (B) EMPLACEMENT:11..19
      (D) AUTRES INFORMATIONS:/note= "SEQ ID NO 1 DE 915 A 923"
   (xi) DESCRIPTION DE LA SEQUENCE: SEQ ID NO: 11:
      AGATGCTGTG TACAAGACC 19
(2) INFORMATIONS POUR LA SEQ ID NO: 12:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..17)
      (D) AUTRES INFORMATIONS:/note= "SEQ ID NO 1 COMPLEMENTAIRE DE 795 A 811"
   (xi) DESCRIPTION DE LA SEQUENCE: SEQ ID NO: 12:
      GCCTGGACAA TGATGAC 17
(2) INFORMATIONS POUR LA SEQ ID NO: 13:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..17)
      (D) AUTRES INFORMATIONS:/note= "SEQ ID NO 1 COMPLEMENTAIRE DE 995 A 1011"
   (xi) DESCRIPTION DE LA SEQUENCE: SEQ ID NO: 13:
      TGATGAAGAT AGAGCCC 17
(2) INFORMATIONS POUR LA SEQ ID NO: 14:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..17
      (D) AUTRES INFORMATIONS:/note= "SEQ ID NO 1 DE 204 A 220"
   (xi) DESCRIPTION DE LA SEQUENCE: SEQ ID NO: 14:
      CGGGTCATGG CAGACTC 17
(2) INFORMATIONS POUR LA SEQ ID NO: 15:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..17)
      (D) AUTRES INFORMATIONS:/note= "SEQ ID NO 1 COMPLEMENTAIRE DE 249 A 265"
   (xi) DESCRIPTION DE LA SEQUENCE: SEQ ID NO: 15:
      GTTTGAAGAA GCATTTG 17
(2) INFORMATIONS POUR LA SEQ ID NO: 16:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..17
      (D) AUTRES INFORMATIONS:/note= "SEQ ID NO 1 DE 330 A 346"
      (xi) DESCRIPTION DE LA SEQUENCE: SEQ ID NO: 16:
         CCTGAGTCAG GAGAATC 17
(2) INFORMATIONS POUR LA SEQ ID NO: 17:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT : complément (1..17)
      (D) AUTRES INFORMATIONS:/note= "SEQ ID NO 1 COMPLEMENTAIRE DE 375 A 391"
   (xi) DESCRIPTION DE LA SEQUENCE: SEQ ID NO: 17:
      AGTCTCAGGA ATTCTTC 17
(2) INFORMATIONS POUR LA SEQ ID NO: 18:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..17
      (D) AUTRES INFORMATIONS:/note= "SEQ ID NO 1 DE 503 A 519"
   (xi) DESCRIPTION DE LA SEQUENCE: SEQ ID NO: 18:
      AGCTGACTTT GACATCA 17
(2) INFORMATIONS POUR LA SEQ ID NO: 19:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..17)
      (D) AUTRES INFORMATIONS:/note= "SEQ ID NO 1 COMPLEMENTAIRE DE 587 A 603"
   (xi) DESCRIPTION DE LA SEQUENCE: SEQ ID NO: 19:
      GCGCTGACTC CATATCC 17
(2) INFORMATIONS POUR LA SEQ ID NO: 20:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:13..33
      (D) AUTRES INFORMATIONS:/note= "SEQ ID NO 1 DE 42 A 62"
   (xi) DESCRIPTION DE LA SEQUENCE: SEQ ID NO: 20:
      CGCGGATCCA CCATGGCAGA AGGCAACCAC AGA 33
(2) INFORMATIONS POUR LA SEQ ID NO: 21:
   (i) CARACTERISTIQUES DE LA SEQUENCE:
      (A) LONGUEUR: 39 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (19..39)
      (D) AUTRES INFORMATIONS:/note= "SEQ ID NO 1 COMPLEMENTAIRE DE 1155 A 1175"
   (xi) DESCRIPTION DE LA SEQUENCE: SEQ ID NO: 21:
      GGCTCTAGAC TCGAGTTATC AATTGCCAGG AAAGAGGTA 39
(2) INFORMATIONS POUR LA SEQ ID NO: 22:
   (i) CARACTERISTIQUES DE LA SEQUENCE:
      (A) LONGUEUR: 1310 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: CDS
      (B) EMPLACEMENT:104..1195
   (xi) DESCRIPTION DE LA SEQUENCE: SEQ ID NO: 22:
   GAACAACGTG GCTGGACAAA CATCTATCCA GACCCTAGTA CCTAATACGG ATCAAAAGTC       60
   GACCAGTGTA AAAAAAGACA ACCACAAAAA AAAAACAGTT AAG ATG TTG GAA TAC        115
   CTG GGC AAA GAT GTT CTT CAT GGT GTT TTT AAT TAT TTG GCA AAA CAC        163
   GAT GTT CTG ACA TTG AAG GAA GAG GAA AAG AAA AAA TAT TAT GAT GCC        211
   AAA ATT GAA GAC AAG GCC CTG ATC TTG GTA GAC TCT TTG CGA AAG AAT        259
   CGC GTG GCT CAT CAA ATG TTT ACC CAA ACA CTT CTC AAT ATG GAC CAA        307
   AAG ATC ACC AGT GTA AAA CCT CTT CTG CAA ATC GAG GCT GGA CCA CCT        355
   GAG TCA GCA GAA TCT ACA AAT ATA CTC AAA CTT TGT CCT CGT GAA GAA        403
   TTC CTG AGA CTG TGT AAA AAA AAT CAT GAT GAG ATC TAT CCA ATA AAA        451
   AAG AGA GAG GAC CGC AGA CGC CTG GCT CTC ATC ATA TGC AAT ACA AAG        499
   TTT GAT CAC CTG CCT GCA AGG AAT GGG GCT CAC TAT GAC ATC GTG GGG        547
   ATG AAA AGG CTG CTT CAA GGC CTG GGC TAC ACT GTG GTT GAC GAA AAG        595
   AAT CTC ACA GCC AGG GAT ATG GAG TCA GTG CTG AGG GCA TTT GCT GCC        643
   AGA CCA GAG CAC AAG TCC TCT GAC AGC ACG TTC TTG GTA CTC ATG TCT        691
   CAT GGC ATC CTA GAG GGA ATC TGC GGA ACT GCG CAT AAA AAG AAA AAA        739
   CCG GAT GTG CTG CTT TAT GAC ACC ATC TTC CAG ATA TTC AAC AAC CGC        787
   AAC TGC CTC AGT CTA AAG GAC AAA CCC AAG GTC ATC ATT GTC CAG GCC        835
   TGC AGA GGT GAA AAA CAT GGG GAA CTC TGG GTC AGA GAC TCT CCA GCA        883
   TCC TTG GCA CTC ATC TCT TCA CAG TCA TCT GAG AAC CTG GAG GCA GAT        931
   TCT GTT TGC AAG ATC CAC GAG GAG AAG GAC TTC ATT GCT TTC TGT TCT        979
   TCA ACA CCA CAT AAC GTG TCC TGG AGA GAC CGC ACA AGG GGC TCC ATC       1027
   TTC ATT ACG GAA CTC ATC ACA TGC TTC CAG AAA TAT TCT TGC TGC TGC 1075
   CAC CTA ATG GAA ATA TTT CGG AAG GTA CAG AAA TCA TTT GAA GTT CCA 1123
   CAG GCT AAA GCC CAG ATG CCC ACC ATA GAA CGA GCA ACC TTG ACA AGA 1171
   GAT TTC TAC CTC TTT CCT GGC AAT TGAAAATGAA ACCACAGGCA GCCCAGCCCT 1225
   CCTCTGTCAA CATCAAAGAG CACATTTACC AGTATAGCTT GCATAGTCAA TATTTGGTAT 1285
   TTCAATAAAA GTAAAGACTG TATCT 1310
(2) INFORMATIONS POUR LA SEQ ID NO: 23:
   (i) CARACTERISTIQUES DE LA SEQUENCE:
      (A) LONGUEUR: 364 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 23:
(2) INFORMATIONS POUR LA SEQ ID NO: 24:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..19
      (D) AUTRES INFORMATIONS:/note= "SEQ ID NO 22 DE 685 A 703"
      (xi) DESCRIPTION DE LA SEQUENCE: SEQ ID NO: 24:
         CATGTCTCAT GGCATCCTA 19
(2) INFORMATIONS POUR LA SEQ ID NO: 25:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..20
      (D) AUTRES INFORMATIONS:/note= "SEQ ID NO 22 DE 712 A 731"
   (xi) DESCRIPTION DE LA SEQUENCE: SEQ ID NO: 25:
      CTGCGGAACT GCGCATAAAA 20
(2) INFORMATIONS POUR LA SEQ ID NO: 26:
   (i) CARACTERISTIQUES DE LA SEQUENCE:
      (A) LONGUEUR: 35 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (16..35)
      (D) AUTRES INFORMATIONS:/note= "SEQ ID NO 22 COMPLEMENTAIRE DE 1229 A 1248"
   (xi) DESCRIPTION DE LA SEQUENCE: SEQ ID NO: 26:
      GGCTCTAGAC TCGAGGTGCT CTTTGATGTT GACAG 35
(2) INFORMATIONS POUR LA SEQ ID NO: 27:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..20)
      (D) AUTRES INFORMATIONS:/note= "SEQ ID No 22 COMPLEMENTAIRE DE 939 A 958"
   (xi) DESCRIPTION DE LA SEQUENCE: SEQ ID NO: 27:
      CTTCTCCTCG TGGATCTTGC 20
(2) INFORMATIONS POUR LA SEQ ID NO: 28:
   (i) CARACTERISTIQUES DE LA SEQUENCE:
      (A) LONGUEUR: 16 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..16
      (D) AUTRES INFORMATIONS:/note= "SEQ ID No 22 DE 124 A 139"
   (xi) DESCRIPTION DE LA SEQUENCE: SEQ ID NO: 28:
      AGATGTTCTT CATGGT 16
(2) INFORMATIONS POUR LA SEQ ID NO: 29:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..17
      (D) AUTRES INFORMATIONS:/note= "SEQ ID NO 22 DE 290 A 306"
   (xi) DESCRIPTION DE LA SEQUENCE: SEQ ID NO: 29:
      CTTCTCAATA TGGACCA 17
(2) INFORMATIONS POUR LA SEQ ID NO: 30:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..17
      (D) AUTRES INFORMATIONS:/note= "SEQ ID NO 22 DE 472 A 488"
   (xi) DESCRIPTION DE LA SEQUENCE: SEQ ID NO: 30:
      CCTGGCTCTC ATCATAT 17
(2) INFORMATIONS POUR LA SEQ ID NO: 31:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..18
      (D) AUTRES INFORMATIONS:/note= "SEQ ID NO 22 DE 634 A 651"
   (xi) DESCRIPTION DE LA SEQUENCE: SEQ ID NO: 31:
      ATTTGCTGCC AGACCAGA 18
(2) INFORMATIONS POUR LA SEQ ID NO: 32:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..19
      (D) AUTRES INFORMATIONS:/note= "SEQ ID NO 22 DE 833 A 851"
   (xi) DESCRIPTION DE LA SEQUENCE: SEQ ID NO: 32:
      GCCTGCAGAG GTGAAAAAC 19
(2) INFORMATIONS POUR LA SEQ ID NO: 33:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..19
      (D) AUTRES INFORMATIONS:/note= "SEQ ID NO 22 DE 1020 A 1038"
   (xi) DESCRIPTION DE LA SEQUENCE: SEQ ID NO: 33:
      GCTCCATCTT CATTACGGA 19
(2) INFORMATIONS POUR LA SEQ ID NO: 34:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..17)
      (D) AUTRES INFORMATIONS:/note= "SEQ ID NO 22 COMPLEMENTAIRE DE 1094 A 1110"
   (xi) DESCRIPTION DE LA SEQUENCE: SEQ ID NO: 34:
      GATTTCTGTA CCTTCCG 17
(2) INFORMATIONS POUR LA SEQ ID NO: 35:
   (i) CARACTERISTIQUES DE LA SEQUENCE:
      (A) LONGUEUR: 16 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..16)
      (D) AUTRES INFORMATIONS:/note= "SEQ ID NO 22 COMPLEMENTAIRE DE 715 A 730"
   (xi) DESCRIPTION DE LA SEQUENCE: SEQ ID NO: 35:
      TTTATGCGCA GTTCCG 16
(2) INFORMATIONS POUR LA SEQ ID NO: 36:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..18)
      (D) AUTRES INFORMATIONS:/note= "SEQ ID NO 22 COMPLEMENTAIRE DE 521 A 538"
   (xi) DESCRIPTION DE LA SEQUENCE: SEQ ID NO: 36:
      GTCATAGTGA GCCCCATT 18
(2) INFORMATIONS POUR LA SEQ ID NO: 37:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..17)
      (D) AUTRES INFORMATIONS:/note= "SEQ ID NO 22 COMPLEMENTAIRE DE 385 A 401"
   (xi) DESCRIPTION DE LA SEQUENCE: SEQ ID NO: 37:
      CTTCACGAGG ACAAAGT 17
(2) INFORMATIONS POUR LA SEQ ID NO: 38:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..17)
      (D) AUTRES INFORMATIONS:/note= "SEQ ID NO 22 COMPLEMENTAIRE DE 237 A 253"
   (xi) DESCRIPTION DE LA SEQUENCE: SEQ ID NO: 38:
      TCGCAAAGAG TCTACCA 17
(2) INFORMATIONS POUR LA SEQ ID NO: 39:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:10..33
      (D) AUTRES INFORMATIONS:/note= "SEQ ID NO 22 DE 101 A 124"
   (xi) DESCRIPTION DE LA SEQUENCE: SEQ ID NO: 39:
      CGCGGATCCA AGATGTTGGA ATACCTGGGC AAA 33
(2) INFORMATIONS POUR LA SEQ ID NO: 40:
   (i) CARACTERISTIQUES DE LA SEQUENCE:
      (A) LONGUEUR: 68 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:46..68
      (D) AUTRES INFORMATIONS:/note= "SEQ ID NO 22 DE 305 A 327"
   (xi) DESCRIPTION DE LA SEQUENCE: SEQ ID NO: 40:
      CGCGGATCCA CCATGGCTTC TATGACAGGA GGTCAACAAA TGGGACAAAA GATCACCAGT 60
      GTAAAACC 68
(2) INFORMATIONS POUR LA SEQ ID NO: 41:
   (i) CARACTERISTIQUES DE LA SEQUENCE:
      (A) LONGUEUR: 36 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:complement (1..16)
      (D) AUTRES INFORMATIONS:/note= "SEQ ID NO 22 COMPLEMENTAIRE DE 838 A 853"
   (ix) CARACTERISTIQUE:
      (A) NOM/CLE: misc_feature
      (B) EMPLACEMENT:complement (18..36)
      (D) AUTRES INFORMATIONS:/note= "SEQ ID NO 22 COMPLEMENTAIRE DE 818 A 836"
   (xi) DESCRIPTION DE LA SEQUENCE: SEQ ID NO: 41:
      ATGTTTTTCA CCTCTGGAGG CCTGGACAAT GATGAC 36
(2) INFORMATIONS POUR LA SEQ ID NO: 42:
   (i) CARACTERISTIQUES DE LA SEQUENCE:
      (A) LONGUEUR: 36 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "OLIGONUCLEOTIDE"
   (ix) CARACTERISTIQUE:
      (A) NOM/CLE: misc_feature
      (B) EMPLACEMENT:1..19
      (D) AUTRES INFORMATIONS:/note= "SEQ ID NO 22 DE 818 A 836"
   (ix) CARACTERISTIQUE:
      (A) NOM/CLE: misc_feature
      (B) EMPLACEMENT:21..36
      (D) AUTRES INFORMATIONS:/note= "SEQ ID NO 22 DE 838 A 853"
   (xi) DESCRIPTION DE LA SEQUENCE: SEQ ID NO: 42:
      GTCATCATTG TCCAGGCCTC CAGAGGTGAA AAACAT 36

## Revendications

1. sequence d'ADN codant pour un polypeptide ayant une activité protéase et capable d'induire l'apoptose et consistant en la séquence nucléotidique de la séquence SEQ ID N° 22.

2. séquence d'ADN selon la revendication 1 commencant au nucléotide 104 et se terminant au nucléotide 1195 de la séquence SEQ ID N° 22.

3. Polypeptide humain ayant une activité protéase et capable d'induire l'apoptose et consistant en la séquence en acides aminés de la séquence SEQ ID N° 23 et désigné protéine Ty.

4. Procédé qui comprend l'expression de la protéine Ty dans une cellule hôte transformée par un ADN codant pour la séquence en acides aminés de la séquence SEQ ID N° 23.

5. ADN consistant en la séquence nucléotidique SEQ ID N° 1.

6. Composition pharmaceutique renfermant à titre de médicament le polypeptide selon la revendication 3.

7. A titre de médicament le polypeptide selon la revendication 3.

8. Composition pharmaceutique renfermant comme principe actif un médicament selon la revendication 7.

9. Composition pharmaceutique selon la revendication 8 pour moduler la production d'IL-1 béta.

10. Composition pharmaceutique selon la revendication 6 pour moduler l'apoptose.

11. Utilisation du polypeptide selon la revendication 3 pour la fabrication d'un médicament pour le traitement des maladies autoimmunes.

12. Utilisation du polypeptide selon la revendication 3 ou du polypeptide de séquence SEQ ID N° 2 pour la fabrication d'un médicament pour la cicatrisation des plaies.

## Claims

1. DNA sequence encoding a polypeptide having a protease activity and capable of inducing apoptosis and consisting of the nucleotide sequence of the sequence SEQ ID No. 22.

2. DNA sequence according to Claim 1, beginning at nucleotide 104 and ending at nucleotide 1195 of the sequence SEQ ID No. 22.

3. Human polypeptide having a protease activity and capable of inducing apoptosis and consisting of the amino acid sequence of the sequence SEQ ID No. 23 and denoted protein Ty.

4. Method which comprises the expression of the protein Ty in a host cell transformed with a DNA encoding the amino acid sequence of the sequence SEQ ID No. 23.

5. DNA consisting of the nucleotide sequence SEQ ID No. 1.

6. Pharmaceutical composition containing, as medicinal product, the polypeptide according to Claim 3.

7. As a medicinal product, the polypeptide according to Claim 3.

8. Pharmaceutical composition containing, as active ingredient, a medicinal product according to Claim 7.

9. Pharmaceutical composition according to Claim 8, for modulating IL-1 beta production.

10. Pharmaceutical composition according to Claim 6, for modulating apoptosis.

11. Use of the polypeptide according to Claim 3, for the manufacture of a medicinal product for the treatment of autoimmune diseases.

12. Use of the polypeptide according to Claim 3 or of the polypeptide of sequence SEQ ID No. 2, for the manufacture of a medicinal product for wound healing.

## Patentansprüche

1. DNA-Sequenz, die für ein Polypeptid kodiert, das eine Proteaseaktivität aufweist und fähig ist, den Zelltod zu induzieren, und die aus der Nukleotidsequenz der Sequenz SEQ ID Nr. 22 besteht.

2. DNA-Sequenz nach Anspruch 1, die bei Nukleotid 104 der Sequenz SEQ ID Nr. 22 beginnt und bei Nukleotid 1195 dieser Sequenz aufhört.

3. Menschliches Polypeptid, das eine Proteaseaktivität aufweist und fähig ist, den Zelltod zu induzieren, und die aus der Aminosäuresequenz der Sequenz SEQ ID Nr. 23 besteht und Ty-Protein genannt wird.

4. Verfahren, umfassend die Expression des Ty-Proteins in einer Wirtszelle, die mit einer DNA, die für die Aminosäuresequenz der Sequenz SEQ ID Nr. 23 kodiert, transformiert ist.

5. DNA, die aus der Nukleotidsequenz SEQ ID Nr. 1 besteht.

6. Pharmazeutische Zusammensetzung, umfassend das Polypeptid nach Anspruch 3 als eine Arznei.

7. Polypeptid nach Anspruch 3 als eine Arznei.

8. Pharmazeutische Zusammensetzung, die als Wirkstoff eine Arznei nach Anspruch 7 umfaßt.

9. Pharmazeutische Zusammensetzung nach Anspruch 8 zur Modulation der Produktion von IL-1 Beta.

10. Pharmazeutische Zusammensetzung nach Anspruch 6. zur Modulation des Zelltods.

11. Verwendung des Polypeptids nach Anspruch 3 zur Herstellung einer Arznei für die Behandlung von Autoimmunerkrankungen.

12. Verwendung des Polypeptids nach Anspruch 3 oder des Polypeptids der Sequenz SEQ ID Nr. 2 zur Herstellung einer Arznei für die Wundheilung.
